# EUROPEAN PATENT APPLICATION

(11) **EP 3 270 263 A1**
(43) Date of publication of application: **17.01.2018**
(21) Application number: 17179680.8
(22) Date of filing: 05.07.2017
(51) Int. Cl.: G06F 3/01, A61B 5/16

(54) **INFORMATION PROCESSING APPARATUS FOR PRESENTING CONTENT, METHOD FOR CONTROLLING THE SAME, AND CONTROL PROGRAM**

(30) Priority: 15.07.2016 JP 2016140703; 18.01.2017 JP 2017006969
(71) Applicant: Panasonic Intellectual Property Management Co., Ltd., Osaka-shi, Osaka 540-6207 (JP)
(72) Inventor: OKUMURA, Yasuaki, Osaka-shi, Osaka 540-6207 (JP); YANAGAWA, Hiroto, Osaka-shi, Osaka 540-6207 (JP); OHNO, Yumiko, Osaka-shi, Osaka 540-6207 (JP); AOKI, Yuichi, Osaka-shi, Osaka 540-6207 (JP); NAKATA, Mikiya, Osaka-shi, Osaka 540-6207 (JP); ASAI, Akira, Osaka-shi, Osaka 540-6207 (JP)
(74) Representative: Grünecker Patent- und Rechtsanwälte PartG mbB

(57) **Abstract**

An information processing apparatus includes a communication interface that receives biological data regarding a user, a memory that stores the biological data, and a processor that determines a type of feeling experienced by the user in a certain period using the biological data. The processor estimates an event that has occurred to the user in the certain period, stores, in a database, feeling history information in which the certain period, feeling type information indicating the type of feeling, and event information indicating the estimated event are associated with one another, generates, if the feeling type information indicates a feeling of pleasure, image data using the associated event information, and outputs a notification for displaying the image data to a terminal device used by the user.

## Description

### BACKGROUND

### 1. Technical Field

The present disclosure relates to a technique for presenting content to help a user control his/her feelings on the basis of psychology.

### 2. Description of the Related Art

A technique for estimating a user's feeling state on the basis of biological data obtained using a biological sensor and creating recommendations as to a user's future actions on the basis of a history in which feelings experienced by the user and the user's actions at those times are associated with each other has been proposed (refer to Japanese Unexamined Patent Application Publication (Translation of PCT Application) No. 2015-505702).

### SUMMARY

In one general aspect, the techniques disclosed here feature an information processing apparatus including a communication interface that receives biological data obtained from a user, a memory that stores the biological data received by the communication interface, and a processor that determines a type of feeling experienced by the user in a certain period using the biological data. The processor estimates an event that has occurred to the user in the certain period, stores, in a database, feeling history information in which the certain period, feeling type information indicating the type of feeling, and event information indicating the estimated event are associated with one another, generates, if the feeling type information stored in the database indicates a feeling of pleasure, image data for helping the user recall the event using the event information associated with the feeling type information, and outputs a notification for displaying the image data to a terminal device used by the user.

The information processing apparatus, a method for processing information, and a program in the present disclosure help a user perform an action supposedly psychologically effective in reducing discomfort in daily life and make it a habit to perform the action.

It should be noted that general or specific aspects may be implemented as a system, a method, an integrated circuit, a computer program, a computer-readable storage medium such as a compact disc read-only memory (CD-ROM), or any selective combination thereof.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### BRIEF DESCRIPTION OF THE DRAWINGS

Fig. 1 is a schematic diagram illustrating an outline of a content presentation system according to a first embodiment;
Fig. 2 is a block diagram illustrating the configuration of the content presentation system according to the first embodiment;
Fig. 3 is a sequence diagram illustrating an example of transmission of data between components of the content presentation system according to the first embodiment;
Fig. 4 is a flowchart illustrating the operation of a server in the content presentation system;
Fig. 5 is a flowchart illustrating a series of steps of a process performed on an analog signal of biological data;
Fig. 6 is a diagram illustrating an example of the configuration of a feeling log database;
Fig. 7 is a flowchart illustrating the operation of a mobile information terminal in the content presentation system;
Fig. 8 is a block diagram illustrating the configuration of a content presentation system according to a second embodiment;
Fig. 9 is a diagram illustrating an example of the data structure of a wakeup feeling log database;
Fig. 10 is a diagram illustrating an example of the data structure of a category distribution log;
Fig. 11A is a flowchart illustrating an operation cycle of the entirety of the content presentation system according to the second embodiment;
Fig. 11B is a flowchart illustrating a procedure of collection of data performed by the content presentation system according to the second embodiment;
Fig. 11C is a flowchart illustrating a procedure of presentation of content performed by the content presentation system according to the second embodiment; and
Fig. 12 is a diagram illustrating an example of an operation screen displayed in a step of presentation of content in each of the embodiments.

### DETAILED DESCRIPTION

### Underlying Knowledge Forming Basis of Present Disclosure

With the technique disclosed in Japanese Unexamined Patent Application Publication (Translation of PCT Application) No. 2015-505702, for example, a user who has received recommendations as to future actions from an apparatus that achieves the technique needs to make plans and preparations in order to perform or continue to perform the recommended actions. That is, it is easy to use the technique when the user can make plans in order to perform recommended special actions or when the user performs a recommended action just once for the purpose of recreation. When the user is stressed out and has a feeling of discomfort such as irritation, depression, or tension due to a daily routine such as work or housework, however, it is difficult to improve the user's feeling state without changing the user's daily life.

The present disclosure provides a content presentation system and the like capable of helping the user perform an action supposedly psychologically effective in reducing discomfort in daily life and make it a habit to perform the action.

The present inventors focused upon one of methods for increasing a feeling of happiness proposed in positive psychology, which is a branch of psychology, with the aim of reducing discomfort experienced by the user in his/her daily life. In the method, the user tries to recall happy events that have occurred that day (hereinafter referred to as "recall of feelings of pleasure") before going to sleep. It is believed that, as a result, the user can change his/her impression of that day in a good way and, by making it a habit to recall feelings of pleasure, get an enduring feeling of happiness.

Since the user need not make plans in this method, it is easier for the user to recall feelings of pleasure in his/her daily cycle than to actually perform an action that evokes a positive feeling. If the user can make it a habit to recall feelings of pleasure, the user will be able to control feelings evoked in his/her daily life. The present inventors, however, have also found that it can be difficult for the user to make it a habit to recall feelings of pleasure using this method when, for example, happy events to be recalled are obscured by other miscellaneous memories of that day or when the user forgets to recall feelings of pleasure due to fatigue or preparation for a next day. In view of this, the present inventors have arrived at the following techniques relating to presentation of content for helping the user continue to perform an action for controlling his/her feelings on the basis of psychology.

An information processing apparatus according to an aspect of the present disclosure includes a communication interface that receives biological data obtained from a user, a memory that stores the biological data received by the communication interface, and a processor that determines a type of feeling experienced by the user in a certain period using the biological data. The processor estimates an event that has occurred to the user in the certain period, stores, in a database, feeling history information in which the certain period, feeling type information indicating the type of feeling, and event information indicating the estimated event are associated with one another, generates, if the feeling type information stored in the database indicates a feeling of pleasure, image data for helping the user recall the event using the event information associated with the feeling type information, and outputs a notification for displaying the image data to a terminal device used by the user.

As a result, the user is prompted to watch image content in order to recall an event that has evoked a feeling of pleasure.

For example, the processor may obtain information indicating the user's bedtime set by the user and, when the user's bedtime has come, output the notification to the terminal device.

As a result, the user is prompted to recall a feeling of pleasure before going to sleep.

In addition, for example, the processor may obtain information for identifying the user's working period and give priority to image data for helping the user recall an event that has occurred outside the user's working period over image data for helping the user recall an event that has occurred in the user's working period in the outputting to the terminal device.

As a result, it becomes possible to avoid making the user recall an event that has occurred in a period likely to evoke a feeling of discomfort when the user tries to recall a feeling of pleasure.

In addition, for example, the user's feeling may be estimated on the basis of biological data regarding the user measured after the user watches, on the terminal device, the image data for helping the user recall an event that has occurred outside the user's working period for a certain period. If the user's feeling does not decrease after the certain period in terms of a feeling of pleasure, image data for helping the user recall an event that has evoked a feeling of pleasure in the user's working period may be output to the terminal device.

As a result, when the user has successfully made it a habit to recall a feeling of pleasure, the user is prompted to recall an event that has occurred in a period likely to evoke a feeling of discomfort in order to enable the user to control his/her feelings even in the period likely to evoke a feeling of discomfort.

In addition, for example, the processor may refer to the database, create a list of pieces of content arranged in order of intensity of feelings of pleasure evoked by events relating to image data to be presented, output a notification for displaying the list of the pieces of content to the terminal device, and, after the user selects a piece of image data included in the list of the pieces of content, display the piece of content on the terminal device,

As a result, the user tends to recognize the recall of a feeling of pleasure before going to sleep as an enjoyable daily routine.

In addition, for example, the processor may obtain external image data uploaded by another user, who is a user other than the foregoing user, to a location accessible by the processor, refer to the database, select a piece of the external image data likely to evoke a feeling of pleasure from the user, and output a notification for displaying the selected piece of the external image data to the terminal device used by the user.

As a result, for example, enriched image content can be presented even to a busy user and is used to help the user recall a feeling of pleasure.

In addition, for example, the communication interface may receive biological data regarding another user, who is a user other than the foregoing user. The memory may store the biological data regarding the other user received by the communication interface. The processor may determine a type of feeling experienced by the other user in a certain period, estimate an event that has occurred to the other user in the certain period, store, in a database, feeling history information in which the certain period, feeling type information indicating the type of feeling experienced by the other user, and event information indicating the event that has occurred to the other user are associated with one another, generate, if the feeling type information regarding the other user stored in the database indicates a feeling of pleasure, image data indicating the event that has evoked the feeling of pleasure from the other user using the event information associated with the feeling type information regarding the other user, and display the image data on the terminal device used by the foregoing user.

As a result, enriched image content can be presented to the user and is used to help the user recall a feeling of pleasure.

In addition, for example, the processor may perform the determination as to a type of feeling, the estimation of an event, the storing in the database, the generation of image data, and the outputting of a notification to the terminal device in an active period, which is a period in which the user is awake. The generated image data may be displayed to help the user recall an event associated with feeling type information indicating a feeling of pleasure in the database among events identified in the active period in which the generation is performed.

As a result, the user is prompted to recall a feeling of pleasure in his/her daily cycle immediately before the user goes to sleep in a manner supposedly psychologically effective in reducing discomfort.

In addition, for example, the processor may also estimate, in the active period, intensity of the user's feeling of pleasure on the basis of biological data regarding the user obtained immediately after the user gets up, select a content presentation criterion on the basis of the estimated intensity of the user's feeling of pleasure, select, from among at least one pleasurable event identified in the active period, an event to be recalled by the user by displaying the image data such that the selected content presentation criterion is satisfied, and generate, as the image data, image data for helping the user recall the selected event to be recalled.

As a result, whether image content presented before the user goes to sleep was appropriate to the user is checked on the basis of the user's feeling immediately after the user gets up. A result of the check is used for feedback, and next image content is presented in accordance with a certain criterion in order to appropriately help the user recall a feeling of pleasure.

As a specific example of the criterion, the processor may also classify the estimated event into a category, refer to the selected content presentation criterion, which specifies a number of events to be recalled for each category, select, from among the at least one pleasurable event identified in the active period, events to be recalled corresponding to the number of events to be recalled specified for each category in the selected content presentation criterion on the basis of the category into which the estimated event has been classified, and generate image data for helping the user recall the selected events to be recalled. As a result, if a category of an event to be recalled by the user through presentation of image content is inappropriate, image content for helping the user recall an event belonging to a category that has been appropriate before is presented next time.

In addition, for example, the processor may also determine a type of feeling that is being experienced by the user on the basis of biological data regarding the user obtained while the image data is being presented to the user and, if the determined type of feeling is a feeling of discomfort, stop presenting the image data to the user.

As a result, even if image content presented on the basis of an incorrect result of estimation of a feeling evokes a feeling of discomfort from the user, an adverse effect upon the user with respect to the recall of a feeling of pleasure is suppressed.

In addition, for example, if a plurality of pieces of the image data are generated, the processor may also select, after stopping presenting the image data to the user, one of the plurality of pieces of the image data other than the image data that has been presented and present the selected piece of the image data to the user.

As a result, even if image content presented on the basis of an incorrect result of estimation of a feeling evokes a feeling of discomfort from the user, it is possible to continue to help the user recall a feeling of pleasure.

In addition, a method for controlling an information processing apparatus according to an aspect of the present disclosure is a method for controlling an information processing apparatus, which performs operations including receiving biological data obtained from a user through a communication network, storing the received biological data in a memory, determining a type of feeling experienced by the user in a certain period using the received biological data, estimating an event that has occurred to the user in the certain period, storing, in a database, feeling history information in which the certain period, feeling type information indicating the type of feeling, and event information indicating the estimated event are associated with one another, generating, if the feeling type information stored in the database indicates a feeling of pleasure, image data for helping the user recall the event using the event information associated with the feeling type information, and outputting a notification for displaying the image data to a terminal device used by the user.

As a result, the information processing apparatus performs a method for prompting the user to recall an event that has evoked a feeling of pleasure.

It should be noted that general or specific aspects may be implemented as, in addition to the above-described system and method, an integrated circuit, a computer program, a computer-readable recording medium such as a compact disc read-only memory (CD-ROM), or any selective combination thereof.

Embodiments will be specifically described hereinafter with reference to the drawings. The following embodiments are general or specific examples. Values, components, arrangement positions and connection modes of the components, steps, the order of the steps, and the like mentioned in the following embodiments are examples for describing the concept of the present disclosure, and do not limit the present disclosure.

Among the components described in the following embodiments, ones not included in the independent claims, which define broadest concepts, will be described as arbitrary components.

### First Embodiment

### 1. Configuration

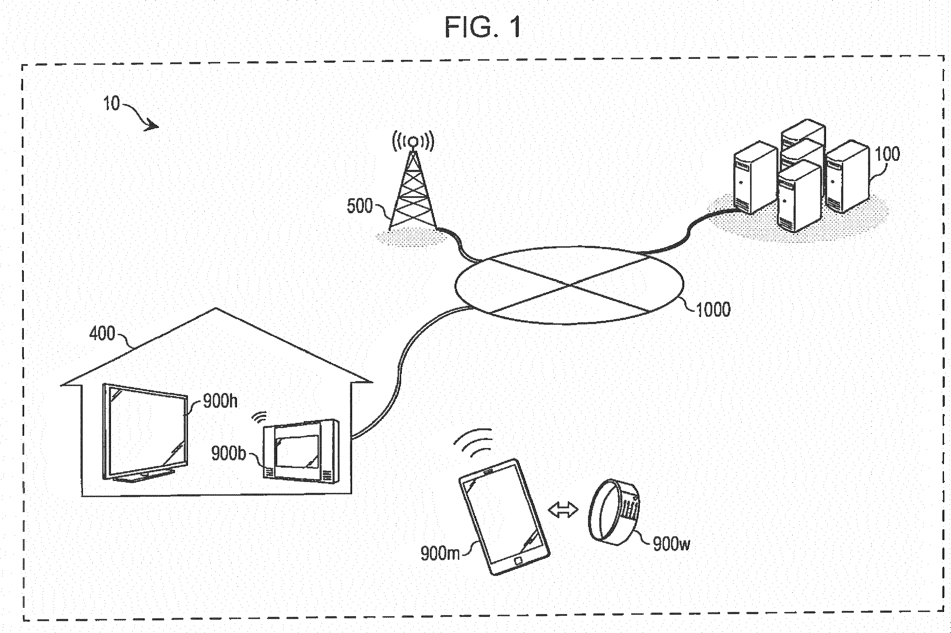

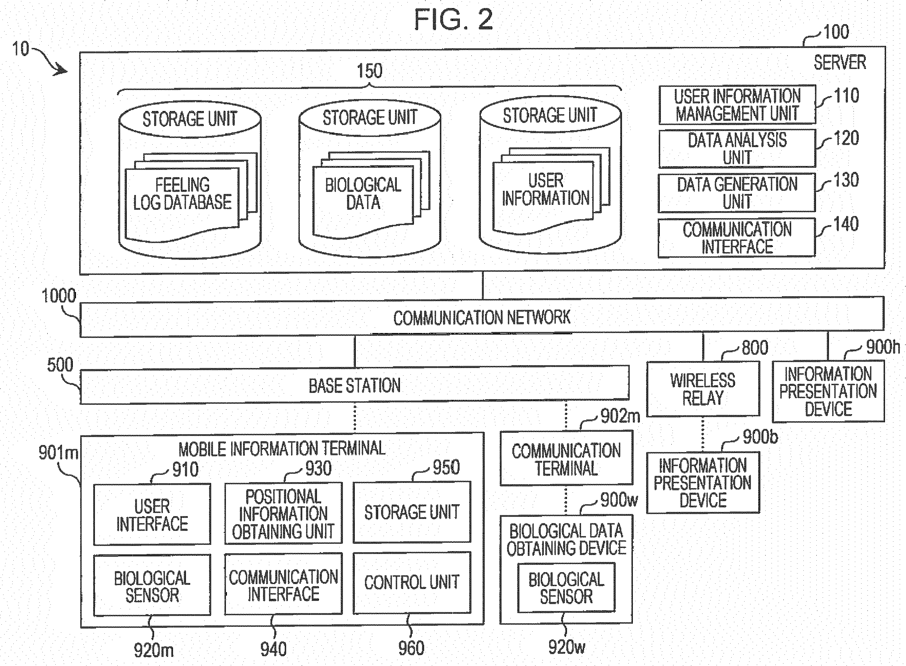

Fig. 1 is a schematic diagram illustrating an outline of a content presentation system that helps a user recall feelings of pleasure according to a first embodiment. Fig. 2 is a block diagram illustrating the configuration of the content presentation system according to the first embodiment. First, the outline of the content presentation system according to the present embodiment will be described with reference to Figs. 1 and 2.

As illustrated in Fig. 1, a content presentation system 10 according to the present embodiment includes a server 100, a mobile information terminal 900m, a biological data obtaining device 900w, an information presentation device 900h, and an information presentation device 900b.

The server 100 communicates data with the mobile information terminal 900m carried by the user of the content presentation system 10 through a communication network 1000 such as the Internet. As illustrated in Fig. 1, the communication network 1000 may include a base station 500.

The server 100 receives data from the biological data obtaining device 900w carried or worn by the user of the content presentation system 10. In Fig. 1, the biological data obtaining device 900w is illustrated as an example of a device that does not have a function of directly connecting to the communication network 1000. The server 100, therefore, receives data from the biological data obtaining device 900w through the mobile information terminal 900m, which functions as a communication terminal 902m illustrated in Fig. 2. The communication terminal 902m may be a device specialized in a communication function, such as a mobile router, instead of the mobile information terminal 900m.

The server 100 also transmits data to the information presentation device 900h or 900b used in a certain place such as a home 400 of the user of the content presentation system 10 through the communication network 1000. For the transmission of data to the information presentation device 900b, a wireless relay 800 illustrated in Fig. 2 is also used along with the communication network 1000.

The content presentation system 10 is thus configured such that the server 100 can communicate data with the devices for collecting data from the user of the content presentation system 10 and the devices for presenting data to the user. The routes described above are an example of routes for communicating data. The routes for communicating data are not limited to this example. The server 100 is preferably in such a state as to be able to receive data constantly and stably from the devices for collecting data while the user is awake (hereinafter referred to as an "active period"; the rest of a day will be referred to as a "sleep period") in his/her daily cycle.

The server 100 is an example of an information processing apparatus according to the present embodiment and achieved by one or more server computers such as ones used for cloud computing. The operation of the server 100 will be described later.

As illustrated in Fig. 2, the server 100 includes storage units 150, a user information management unit 110, a data analysis unit 120, a data generation unit 130, and a communication interface 140.

The storage units 150 are memories that store various pieces of data used to execute functions of the server 100 that will be described later. The storage units 150 are achieved by magnetic storage devices such as hard disk drives or semiconductor storage devices such as flash memories. The various pieces of data stored in the storage units 150 will be described hereinafter. The memories herein refer to a concept including components, devices, and media, which include storages, storing data used by the information processing apparatus.

The user information management unit 110 is a component achieved by a processor (not illustrated) included in the server 100 when the processor executes a program. The user information management unit 110 receives, holds, and updates information for presenting content using data selected or created for each user. This information will be described later. The user information management unit 110 also authenticates the user using this information.

The data analysis unit 120 and the data generation unit 130 are components achieved by the processor included in the server 100 when the processor executes a program. The data analysis unit 120 mainly analyzes data collected from the user. The data generation unit 130 generates data to be presented to the user. The analysis and generation of data will be described later as a part of the operation of the server 100.

The communication interface 140 is a component achieved by a communication module and used for the above-described series of communication operations through the communication network 1000. The communication interface 140 receives the following pieces of biological data obtained from the user.

The biological data obtaining device 900w includes a biological sensor 920w. The biological sensor 920w measures and obtains biological data regarding the user. The biological data herein refers to any kind of data that can be technically analyzed and used to estimate the user's feelings, such as body temperature, pulse, blood pressure, subcutaneous blood flow, a respiration rate, the amount of sweat, constituents of a metabolite such as exhaled air or sweat, a vocal sound, brain waves, eye movement, changes in the pupil, facial expression, or any selective combination thereof. The biological sensor 920w may be any kind of sensor capable of obtaining these pieces of biological data, such as one that measures one of various physical values including heat, light (visible or invisible), electric phenomena, magnetic phenomena, sound, vibration, and movement of an object. In order to constantly obtain the biological data regarding the user, the biological data obtaining device 900w is preferably easily worn by the user for an extended period of time in an active period in the user's daily cycle. The biological data obtained by the biological sensor 920w is transmitted to the server 100, received by the communication interface 140 of the server 100, and recorded in the storage units 150. The biological data obtaining device 900w also includes an output unit (not illustrated) for outputting the biological data. The biological data obtaining device 900w is achieved by a wearable information terminal such as a smartwatch, an activity tracker, or smartglasses. Alternatively, the biological data obtaining device 900w may be one of various wearable or portable medical devices for obtaining biological data capable of outputting data through communication.

As described above, the mobile information terminal 900m illustrated in Fig. 1 can also function as the communication terminal 902m that mediates the communication between the biological data obtaining device 900w and the server 100. The mobile information terminal 900m, however, will be described hereinafter as a mobile information terminal 901 m, which is illustrated in the block diagram of Fig. 2, that mainly achieves functions other than the mediation of communication. The mobile information terminal 901m includes a user interface 910, a biological sensor 920m, a positional information obtaining unit 930, a communication interface 940, a storage unit 950, and a control unit 960. The mobile information terminal 901m is achieved by an information device such as a smartphone or a tablet computer.

The user interface 910 refers to components of the information device used to receive information from the user or present information to the user, such as a touch screen (includes operation screens displayed on the touch screen), various buttons, a camera, a speaker, and a microphone. The user interface 910 also refers to components of the content presentation system 10 used to receive information from the user and present information to the user. Information input using the user interface 910 is transmitted to the server 100 through the communication network 1000. Data transmitted from the server 100 is received by the mobile information terminal 901m through the communication network 1000 and presented to the user through the user interface 910 as content such as an image (a still image or a moving image). The content to be presented is not limited to an image, but may be content including sound or content including sound alone, instead. In the following description, however, the embodiments will be described while taking a case in which the content to be presented is content including an image as an example. An expression "presentation of content" implies that the content to be presented is not limited to content including an image, and an expression "display of image data" is equivalent to "presentation of content including an image", which is an example of the "presentation of content".

As with the biological sensor 920w of the biological data obtaining device 900w, the biological sensor 920m measures and obtains biological data. Description of the biological sensor 920w is omitted here. The content presentation system 10 may include at least either the biological sensor 920w or the biological sensor 920m, and the biological sensor 920w and the biological sensor 920m may be used along with each other if the content presentation system 10 includes both. In the following description, the biological sensor 920m and the biological sensor 920w are not particularly distinguished from each other and will be simply referred to as "biological sensors 920".

The positional information obtaining unit 930 is a global positioning system (GPS) receiver, for example, and obtains positional information regarding the mobile information terminal 901 m. The obtained positional information is transmitted to the server 100 and, for example, used by the server 100 as positional information regarding the user. The positional information obtained by the positional information obtaining unit 930 is not limited to information obtained by a satellite positioning system such as a GPS satellite. The positional information may be information obtainable from an access point of Wi-Fi (registered trademark) to which the mobile information terminal 901 m is connected, instead.

The communication interface 940 is achieved by a communication module used by the mobile information terminal 901m to connect to the communication network 1000. If the mobile information terminal 900m is functioning as the communication terminal 902m, a communication module for communicating with the biological data obtaining device 900w is also included in the communication interface 940.

The storage unit 950 is a memory storing various programs to be executed by the mobile information terminal 901m and information obtained by the mobile information terminal 901m and achieved by a semiconductor storage device such as a flash memory.

The control unit 960 is a component achieved by a processor that executes the various programs to control and operate the components of the mobile information terminal 901m. As a result, data used to help the user recall feelings of pleasure is collected and provided for the server 100, and content for helping the user recall feelings of pleasure provided from the server 100 is presented to the user.

The information presentation devices 900b and 900h are capable of presenting content for helping the user recall feelings of pleasure to the user using content data provided from the server 100. The information presentation devices 900h and 900b (hereinafter referred to as "information presentation devices 900" without particularly distinguishing the two) are achieved by devices such as a personal computer and a television set capable of connecting to the Internet. Alternatively, the information presentation devices 900 may be wearable information terminals including a display, such as smartglasses and a head-mounted display. The content presentation system 10 may include at least either the mobile information terminal 901 m or the information presentation device 900h as a component for presenting content to the user. If the content presentation system 10 includes both the mobile information terminal 901 m and the information presentation device 900h, the user may select one of the two and watch content. An information presentation device 900 is an example of a terminal device used by the user in the present embodiment.

It is recommended to recall feelings of pleasure before a person goes to sleep, that is, at an end of a day. An information presentation device 900, therefore, may be a device set in a place where the user stays at an end of an active period of the user's daily cycle, such as a living room or a bedroom. Alternatively, the information presentation device 900 may be a device set in a bathroom depending on the user's preferences, The information presentation device 900 is not limited to a device used in the user's home 400. A personal computer or the like set in a hotel or the like may be used as the information presentation device 900.

Although the configuration of the content presentation system 10 has been described, the above-described configuration is an example, and the configuration of the content presentation system 10 is not limited to this. For example, although the biological data obtaining device 900w and the mobile information terminal 901m have been described as two different devices for the sake of convenience of description of the functions, these devices may be achieved in any way. The biological data obtaining device 900w may, for example, execute some or all of the functions of the mobile information terminal 901 m and communicate with the server 100 through the communication network 1000 without using the communication terminal 902m. In addition, a person computer that achieves the information presentation device 900h may execute some of the functions of the user interface 910.

Next, the operation of the content presentation system 10 will be described while focusing upon transmission of various pieces of data used in the content presentation system 10.

### 2. Operation

### 2-1. Outline of Operation of Content Presentation System

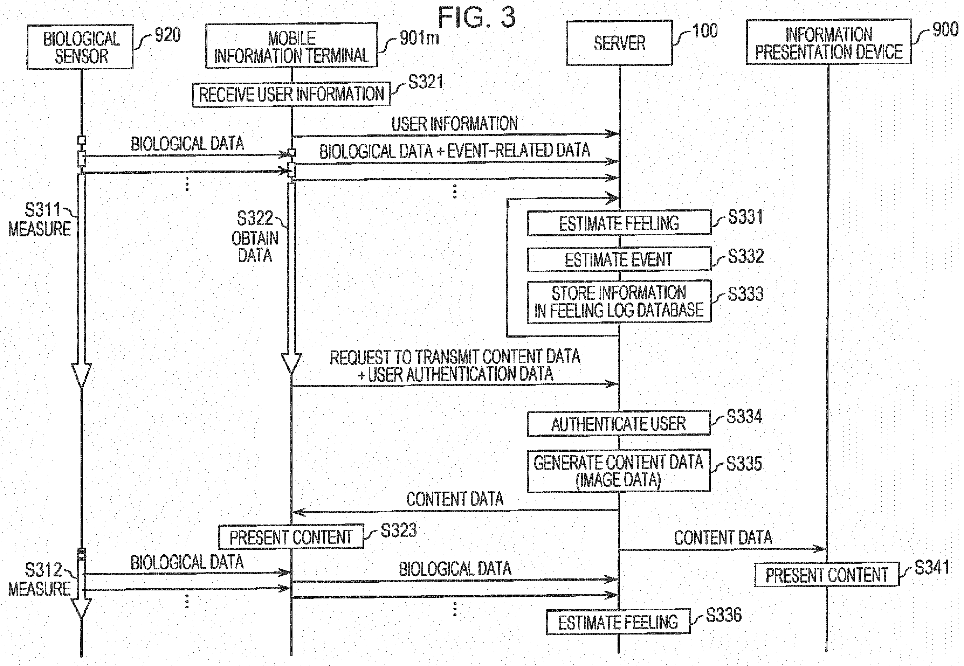

First, an outline of the operation of the content presentation system 10 will be described while focusing upon transmission of data in the content presentation system 10. Fig. 3 is a sequence diagram illustrating an example of transmission of data between the components of the content presentation system 10. In the sequence diagram, biological data regarding the user measured and obtained by a biological sensor 920 is transmitted from the mobile information terminal 901 m to the server 100.

First, the user interface 910 of the mobile information terminal 901 m receives user information regarding the user of the content presentation system 10 (step S321).

The user information includes information used to authenticate the user, such as a user identifier (ID) and a password. In the content presentation system 10, biological data and the like are treated as data unique to each user. Content data to be presented to the user is created for each user on the basis of data unique to the user. If the content presentation system 10 is used by a plurality of users, therefore, various pieces of data need to be managed for each user. Such user information is input, for example, when a user creates an account in order to use the content presentation system 10 for the first time.

The user information may include various settings specified by the user before using the content presentation system 10. A specific example of one of the settings is a setting relating to a timing at which the user recalls feelings of pleasure in the his/her daily cycle. The settings may include, for example, the user's bedtime, which is information relating to a timing at which content data is to be transmitted. The settings specified in the user information may also include information relating to content that the user desires to watch (or not to watch) to help him/her recall feelings of pleasure. Such information includes, for example, settings relating to a type of content (a still image, a moving image, or the like), personal interests (hobbies, keywords, or the like), types of data that can or cannot be used as a source of content, and a place. The user information may also include information necessary to cause the content presentation system 10 to cooperate with a service used by the user on the Internet. The cooperation will be described later in the description of the operation.

The mobile information terminal 901m transmits the input user information to the server 100, and the user information management unit 110 stores the user information in the storage units 150 (refer to Fig. 2).

Next, after the authenticated user begins to use the content presentation system 10, the biological sensor 920 measures and obtains biological data regarding the user in an active state (step S311). The biological data is transmitted to the server 100 through the mobile information terminal 901 m as necessary.

The positional information obtaining unit 930 of the mobile information terminal 901 m obtains current positional information thereon (step S322) and transmits the current positional information thereon to the server 100 as positional information indicating a position of the user. In addition, data regarding a still image or a moving image (hereinafter referred to as "image data" without distinguishing data regarding a still image and a data regarding a moving image) captured by the user using a camera (not illustrated) incorporated into or cooperating with the mobile information terminal 901 m may be transmitted. Data regarding information used in an application used by the user on the mobile information terminal 901m may also be transmitted. The information used in an application is, for example, schedule information indicating the user's schedule used in a schedule application, a keyword input by the user for a search or a uniform resource locator (URL) of a page of a news article accessed by the user in a web browser, or a URL indicating content evaluated or tagged by the user in a social networking service (SNS). The data transmitted from the mobile information terminal 901 m along with the biological data is used by the server 100 to estimate an event that has evoked a feeling from the user. The data used to estimate an event will also be referred to as "event-related data" hereinafter. The mobile information terminal 901m also transmits event-related data to the server 100 as necessary. The data other than the biological data, such as content posted by the user on an SNS or data indicating responses to content posted by others, may also be used to estimate the user's feelings. A URL of a website page accessed by the user may be saved to the storage units 150 and used later to generate data regarding content to be presented to the user.

The server 100 stores the received biological data in the storage units 150. The data analysis unit 120 then uses the biological data to estimate a feeling experienced by the user in a certain period (step S331; a first feeling estimation step). The certain period is, for example, an active period of the user. Alternatively, the certain period may be a part of an active period (a working period, which will be described later, or another period).

The server 100 also estimates an event that has evoked the feeling on the basis of the event-related data received by the data analysis unit 120 (step S332; an event estimation step). An event that has evoked a feeling may be estimated each time a feeling is evoked, regardless of the type of feeling estimated in step S331. Because content is presented to help the user recall feelings of pleasure, however, it is enough to estimate events that have evoked feelings of pleasure (hereinafter referred to as "pleasurable events"). According to Hiromi Akutsu, Yumi Odashima, and Satomi Miya "Change of Positive and Negative Affect by a Stress Task", Iwate University Faculty of Education Departmental Bulletin Paper, Vol. 68 (Feb. 2009), pp.1-8, for example, feelings of pleasure refer to "an emotional state characterized by happiness, well-being, a positive valence, and high activation (arousal)" and more specifically refer to "happiness, joy, satisfaction, curiosity, love., etc."

As specific examples of a method for estimating the user's feeling from biological data and determining whether the user's feeling is a feeling of pleasure, for example, known methods disclosed in Japanese Unexamined Patent Application Publication No. 2012-120206, Japanese Unexamined Patent Application Publication No. 2015-46065, and Japanese Unexamined Patent Application Publication No. 2002-112696 may be used.

In Japanese Unexamined Patent Application Publication No. 2012-120206, a user's psychological state is determined on the basis of the user's heart rate and variation in the user's heart rate. More specifically, the user's heart rate and variation in the user's heart rate are compared with certain thresholds, and if the user's heart rate is higher than the certain threshold and variation in the user's heart rate is larger than the certain threshold, the user's psychological state is determined to be "joy" (that is, a feeling of pleasure).

In Japanese Unexamined Patent Application Publication No. 2015-46065, a level of comfort and a level of arousal are generated from a user's biological sensor values obtained from a biological sensor, and the user's feeling is estimated from a feeling score, which is a combination of the generated level of comfort and level of arousal. The level of comfort and the level of arousal each range from -5 to 5, for example, and if the level of arousal falls within a range of 0 to 2 and the level of comfort falls within a range of 2 to 5, the user is determined to be in a "joyous state" (that is, the user is experiencing a feeling of pleasure).

In Japanese Unexamined Patent Application Publication No. 2002-112696, a feeling (e.g., joy) is recognized using biological parameters obtained from biological sensors, such as blood pressure data, a heart rate, skin temperature, and a frequency spectrum obtained from an electrocardiogram.

Next, the data analysis unit 120 stores information in which data indicating a result of the estimation of a feeling in step S331 and data indicating the estimated event are associated with each other in a database of a feeling log (hereinafter referred to as a "feeling log database") provided in the storage units 150 (step S333). Steps S331 to S333 are repeated while the server 100 is receiving biological data and event-related data in an active period of the user.

As an end of an active period of the user approaches, that is, as a timing at which the user recalls feelings of pleasure approaches, the mobile information terminal 901m transmits a request to transmit content data to the server 100. In this example, user authentication data is also transmitted again.

After the server 100 receives the request and the user authentication data, the user information management unit 110 authenticates the user (step S334). If the user is successfully authenticated, the data generation unit 130 generates content for helping the user recall pleasurable events, that is, data (image data) regarding content such as images indicating the pleasurable events (step S335). A source of the data includes, for example, the event-related data that the server 100 has received from the mobile information terminal 901 m in the active period. If there are a plurality of pleasurable events, some of the plurality of pleasurable events may be selected in accordance with a certain criterion or settings made by the user, and content data relating to the selected pleasurable events may be generated. The data generation unit 130 of the server 100 that generates data regarding content for helping the user recall a pleasurable event identified in step S332 is an example of a data generation device in the present embodiment.

The generated content data is transmitted to the mobile information terminal 901 m. The mobile information terminal 901 m presents the content for helping the user recall pleasurable events to the user using the received content data (step S323). More specifically, for example, a moving image, or a thumbnail thereof, captured by the user in the active period using the mobile information terminal 901m while the user is laughing loud is displayed on the mobile information terminal 901m. The content data may be transmitted to the information presentation device 900 in accordance with an instruction from the user, and the information presentation device 900 may present the content to the user (step S341). The mobile information terminal 901 m and the information presentation device 900 are examples of a terminal device used by the user to watch content.

Before transmitting the content data, the server 100 may output a certain notification to be presented to the user to the mobile information terminal 901m or the information presentation device 900. The notification may, for example, indicate that the image data can be displayed. If the image data corresponds to a plurality of pieces of content, the notification may be an operation screen for selecting one of the plurality of pieces of content.

The biological sensor 920 may measure and obtain biological data regarding the user (step S312) while step S323 or S341 is being performed. As with the biological data obtained in step S311, the biological data obtained in step S312 is transmitted to the server 100 through the mobile information terminal 901 m as necessary. The data analysis unit 120 of the server 100 estimates, on the basis of the biological data, a feeling of the user who is watching the content (step S336; a second feeling estimation step). If a type of feeling estimated in step S336 is a feeling of discomfort, the presented content might be inappropriate as content for helping the user recall feelings of pleasure and adversely affecting the user's feeling. The presentation of the content may therefore be stopped, and if a plurality of pieces of content data have been generated, another piece of data may be presented to the user after the presentation of the content is stopped. As a result, it is possible to continue helping the user recall feelings of pleasure.

When content is presented, the mobile information terminal 901m may receive an instruction to stop presenting the content, an instruction to display another piece of content, an instruction to repeat the presentation of the content, or the like.

The outline of the operation of the content presentation system 10 has been described. In the operation, the steps from the estimation of a feeling by the server 100 (step S331) to the presentation of content by the mobile information terminal 901m (step S323 or S341) are performed in an active period of the user's daily cycle. The content presented to the user in step S323 is content for helping the user recall pleasurable events that have evoked feelings of pleasure from the user in the active period before step S323. The user, therefore, can recall feelings of pleasure in a manner psychologically effective in reducing discomfort by using the content presentation system 10 such that step S323 is performed at an end of an active period of the user's daily cycle, that is, immediately before the user's bedtime. In addition, since presented content helps the user recall pleasurable events, the user can make it a habit to recall feelings of pleasure.

### 2-2. Operation of Server

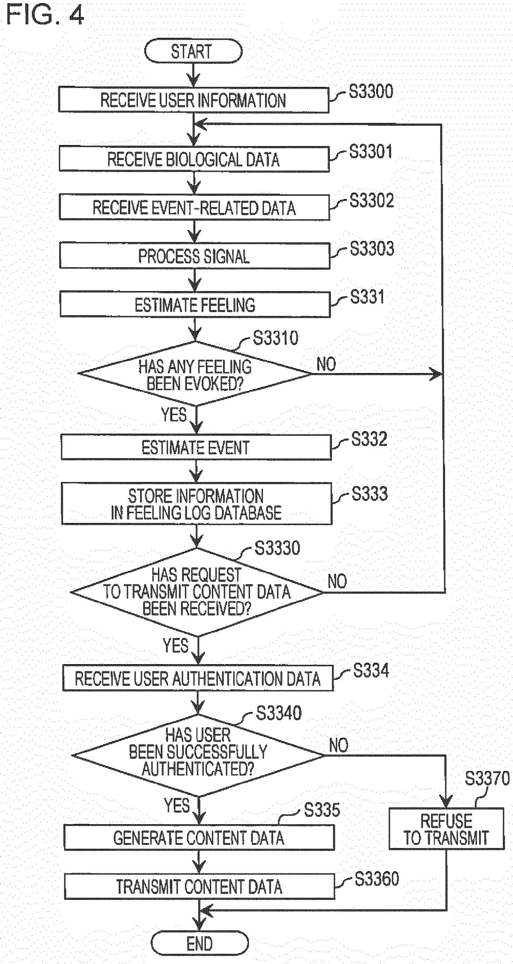

Next, the operation of the server 100 in the content presentation system 10 will be specifically described with reference to a flowchart of Fig. 4 illustrating the operation of the server 100. The following description is a detailed description of the operation of the server 100, whose outline has been described with reference to Fig. 3, and the same steps as those illustrated in Fig. 3 are given the same reference numerals.

First, the server 100 receives user information (step S3300). An account of the user is created using the user information to prepare for management and creation of individualized data.

Next, the server 100 receives biological data measured and obtained by the biological sensor 920 (step S3301). The server 100 also receives event-related data from the mobile information terminal 901m (step S3302).

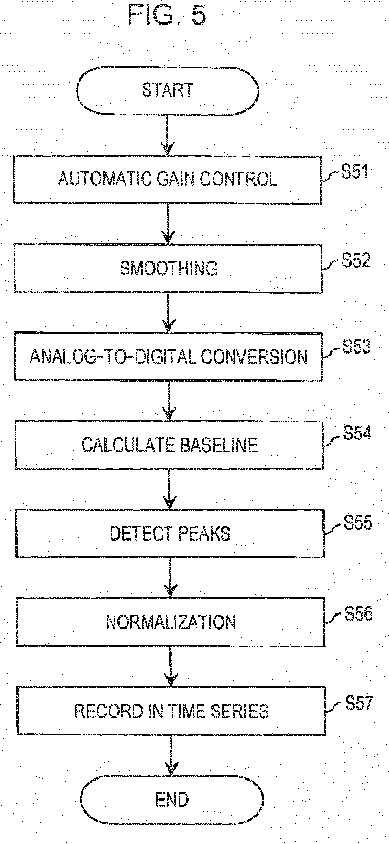

An unprocessed analog signal indicating the biological data obtained by the biological sensor 920 includes a lot of noise and is not suitable for a data analysis. In addition, since the analog signal is obtained through constant measurement, a size thereof is large and the storage units 150 tends to become full. The analog signal, therefore, is subjected to a certain process and then saved and analyzed (step S3303). Fig. 5 is a flowchart illustrating an example of a series of steps of the certain process. The process may be performed not by the server 100 but by the biological sensor 920 or the biological data obtaining device 900w before the analog signal is transmitted. In this case, a traffic load is reduced.

First, an automatic gain control circuit performs automatic gain control on the analog signal (step S51). A low-pass filter then smoothes (removes noise) the analog signal (step S52). Next, the analog signal is converted into data regarding precise discrete values through analog-to-digital conversion (step S53). If there is a significant change in a baseline, the baseline is calculated and subtracted from the original data (step S54). Next, peaks are extracted on the basis of a certain threshold (step S55). As a result, compact data regarding an aggregation of one or more peaks along a time axis is obtained. Next, peak values are normalized as necessary in order to make a dynamic range of the data appropriate, and arranged in a time series (steps S56 and S57).

Next, the data analysis unit 120 of the server 100 estimates feelings using the biological data (step S331; the first feeling estimation step).

Various methods based on past studies can be used to estimate feelings using biological data.

In the method disclosed in Japanese Unexamined Patent Application Publication No. 2012-120206, for example, a psychological state of a user is determined on the basis of the user's heart rate and variation in the user's heart rate. In this method, the user's heart rate and variation in the user's heart rate detected as biological information are compared with the certain thresholds. If the user's heart rate is higher than the certain threshold and variation in the user's heart rate is larger than the certain threshold, the user's psychological state is determined to be "joy" (that is, a feeling of pleasure).

In the method disclosed in Japanese Unexamined Patent Application Publication No. 2015-46065, a user's feeling and the intensity thereof are estimated on the basis of a feeling score generated from biological sensor values of the user obtained using a biological sensor. More specifically, a level of comfort and a level of arousal of the user, which each range from -5 to 5, are generated as a feeling score from biological sensor values. If the level of arousal falls within a range of 0 to 2 and the level of comfort falls within a range of 2 to 5, for example, the user is determined to be in a "joyous state" (that is, the user is experiencing a feeling of pleasure).

The data analysis unit 120 may estimate, using biological data, not only the type of feeling experienced by the user but also the intensity of the feeling based on the level of comfort and the level of arousal.

If the biological data used in step S331 does not indicate any feeling (NO in step S3310), the server 100 performs steps S3301 to S3310 on biological data received next.

If the biological data used in step S3310 indicates any feeling (YES in step S3310), the data analysis unit 120 estimates an event that has evoked the feeling from the user on the basis of the event-related data (step S332). As described above, events searched in step S332 include at least an event that has evoked a feeling of pleasure (pleasurable event).

A pleasurable event is identified using at least any of the positional information indicating the position of the user, the schedule information indicating the user's schedule, and the image data captured by the user included in the event-related data transmitted from the mobile information terminal 901m. More specifically, for example, a pleasurable event may be identified on the basis of information regarding a time at which a feeling of pleasure was evoked and information regarding what the user was doing at that time, which is indicated by the schedule information regarding the user's schedule included in the event-related data. Alternatively, a keyword input by the user for a search with the mobile information terminal 901m or information regarding a news article on the Internet accessed by the user immediately before or after a feeling of pleasure is evoked may be used.

In addition, in the user information registered in advance, a piece of information relating to the user's personal interests may be used to estimate an event. For example, the data analysis unit 120 may collect information on the Internet using information indicating the user's personal interests as keywords and identify an event. If information indicating that one of the user's favorite athletes had made a spectacular showing was obtained immediately before or after the user experienced a feeling of excitement, this event may be estimated as a pleasurable event.

Alternatively, the content presentation system 10 may cooperate with a schedule management service, a blog, an SNS, or another communication service on the Internet and use a time, an activity, positional information, or the like obtained from content posted by the user or content associated with the user by a friend of the user's as information for identifying a pleasurable event.

The event-related data is saved to the storage units 150 as necessary in order to obtain content data later.

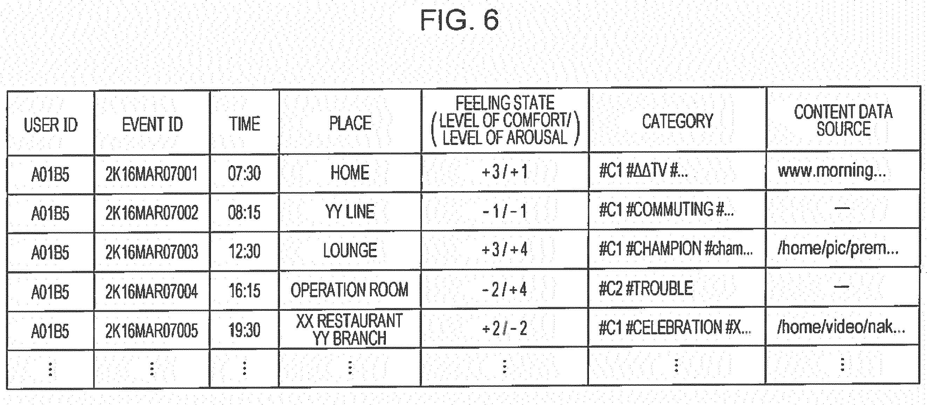

Next, the data analysis unit 120 stores information in which the estimated feeling and the event that has evoked the feeling are associated with each other in the feeling log database provided in the storage units 150 in order to obtain content data later (step S333). Fig. 6 is a diagram illustrating an example of the configuration of the feeling log database.

In the example illustrated in Fig. 6, such pieces of information are recorded in data rows in order of time of evocation of a certain user's feelings (or in order of time of occurrence of events that have caused the certain user's feelings). In each data row, a user ID, an event ID, an evocation time, the user's location, the user's feeling state, a category, and a data source of content are associated with one another from left to light as a record of registered information. Such pieces of information in each record will be referred to as "feeling history information".

The user ID is information for identifying each user and provided to manage an estimated feeling and an event that has evoked the feeling for each user. The user ID is an example of user ID information in the present embodiment.

The event ID is information for identifying each record of registered information and provided to manage information regarding each of identified events.

The time and the location are based on received biological data and event-related data.

The feeling state is a result of an analysis of biological data conducted by the data analysis unit 120 and is information indicating a type of feeling and the intensity of the feeling. In this example, a feeling score generated from biological sensor values using the above-described method is indicated in the field. In this case, the data analysis unit 120 or the data generation unit 130 uses the information in this field to obtain the type of feeling and the intensity of the feeling by referring to a table indicating the type of feeling and the intensity of the feeling corresponding to the feeling score as necessary in the process. In another example, the type of feeling and the intensity of the feeling based on the feeling score may be directly indicated in the field. The information in the "feeling state" field is an example of feeling state information in the present embodiment. In the following description, the feeling state information will be referred to as "feeling type information" as information indicating a type of feeling, and as "feeling intensity information" as information indicating the intensity of a feeling.

Information indicating a category is meta-information regarding an estimated event. For example, the data analysis unit 120 classifies an event into a category on the basis of a period in which the event has occurred and a place included in event-related data and adds information indicating the category to this field. The data analysis unit 120 obtains information identifying a working period (a period from a start time to an end time of the user's office or school) registered by the user as a part of the user information during the creation of the account and classifies an event into a category using the information. In the example illustrated in Fig. 6, an event unrelated to the user's job is classified into category 1 (C1), and an event related to the user's job is classified into category 2 (C2). A hashtag is provided for each category. In addition, information based on various pieces of information included in the event-related data may be added to this category. For example, a word, a hashtag, or the like included by the user in content posted to an SNS or a blog may be added. The information that can be input to the field may be specified for each content presentation system 10 or set by each user. The information in this field is an example of event information, which is information referred to by the data generation unit 130 in a later step.

Information in the "content data source" field is information indicating a location of data referred to in order to create content data relating to an event stored as a part of a record. This information can be, for example, information indicating a location of a media file such as an image included in event-related data transmitted from the mobile information terminal 901 m to the server 100 or a URL on the Internet included in event-related data. Alternatively, the information may be a URL of information obtained by the data analysis unit 120 in step S332 in order to estimate a pleasurable event. The information in this field, too, is an example of the event information referred to by the data generation unit 130 in a later step in order to obtain content data.

As described above, if the user experiences a feeling, feeling state information (feeling type information and feeling intensity information) regarding the feeling and event information indicating an event that has supposedly evoked the feeling are associated with each other and stored in the database as feeling history information. The feeling history information includes information indicating a time at which the user experienced a feeling, a place where the user experienced the feeling, or a category of an event. A certain period (e.g., an active period, a working period, or the like) in which the user experienced the feeling is also indicated on the basis of this information. That is, in the feeling history information, the certain period, the feeling state information, and the event information are associated with one another.

The steps S3301 to S333 are performed each time biological data is received until the mobile information terminal 901 m issues a request to transmit content data (NO in step S3330).

If the server 100 receives a request to transmit content data (YES in step S3330), the server 100 also receives user authentication data (step S334), and the user information management unit 110 authenticates the user (step S3340).

If the user information management unit 110 fails to authenticate the user (NO in step S3340), the server 100 refuses to transmit content data (step S3370). The server 100 may also transmit a notification indicating the refusal to the mobile information terminal 901m.

If the user information management unit 110 successfully authenticates the user (YES in step S3340), the data generation unit 130 then obtains and generates data regarding content to be presented to the user (step S335).

In this step, the data generation unit 130 refers to the feeling log database to obtain content data. For example, the data generation unit 130 refers to the information in the "feeling state" field and selects three pleasurable events that have evoked most intense feelings of pleasure. The data generation unit 130 also refers to the information in the "category" field and, if the user has specified a certain category as content to be presented or excluded a certain category from content to be presented in the user information or the like, follows the user's instruction. After selecting an event for which related content is to be obtained, the data generation unit 130 refers to the information in the "content data source" field and obtains content data, such as images, for helping the user recall the selected pleasurable events.

After obtaining the data, the data generation unit 130 generates data to be presented using the obtained data such that the data is effectively presented on the mobile information terminal 901 m. For example, thumbnails of moving images may be prepared to display a list, or a digest moving image may be generated from a plurality of moving images or still images. In addition, sound effects to be used when a list or a digest is displayed may be added. In addition, information specifying order of presentation may be added. If the user has specified some settings relating to presentation of content, such as settings relating to order of presentation or a type of medium, data may be generated in accordance with the settings.

Lastly, the server 100 transmits the generated content data to the mobile information terminal 901 m (or another terminal device used by the user specified by the user) (step S3360).

Step S3360 may include a two-step procedure including notification after the server 100 completes the preparation of content data and transmission of the content data. The notification prompts the user to display a list of content or play back content on a terminal device such as the mobile information terminal 901m. The terminal device receives a notification indicating the completion of the preparation of content data from the server 100 and outputs the notifcation using one of various notification means including a message and a badge displayed on an icon of an application. When the notification is output, a sound, vibration, light, or any selective combination of these may be used. Alternatively, the notification may be output to a plurality of terminal devices available to the user, and the content data may be transmitted to one that the user has operated. Alternatively, the server 100 may transmit content data to a terminal device specified by the user and then output a notification indicating the completion of the preparation of display or playback of the content data on the terminal device. Such a notification can prevent the user from forgetting to recall feelings of pleasure.

The details of the operation of the server 100 in an active period in the user's daily cycle whose outline has been described with reference to Fig. 3 has been described.

The above description is an example of the operation of the server 100, and the operation of the server 100 in the content presentation system 10 according to the present embodiment is not limited to this.

In the above description, for example, the user is authenticated (steps S334 and S3340) after a request to transmit content data is transmitted in step S3330. The authentication of the user in this stage, however, is not mandatory in the content presentation system 10 according to the present embodiment, If it is likely that the request is coming from an unauthorized user, however, the user is preferably authenticated in this stage. When content data is to be displayed on a terminal device other than the mobile information terminal 901 m that has transmitted biological data or when a terminal device or an Internet protocol (IP) address that the user uses for the first time to present content has accessed the server 100, for example, the user is preferably authenticated in this stage. In addition, when content data is transmitted to a terminal device without the user's instruction as described above, the user may be authenticated immediately before the user uses the transmitted content data.

In addition, in step S3330, the server 100 obtains and generates content data and transmits the content data to the user after receiving a request to transmit content data from the mobile information terminal 901 m. The timing at which the server 100 transmits content data to the user, however, is not limited to this. For example, the server 100 may transmit content data at a time specified by the user in advance (reservation of transmission of content data). In this case, the user can recall feelings of pleasure in accordance with his/her daily cycle or his/her schedule of the day. If the user specifies his/her bedtime, for example, the processor of the server 100 obtains information indicating the user's bedtime and generates content data before the user's bedtime. When the user's bedtime has come, the server 100 outputs the above-described notification to the user's terminal device. The user may specify a time using any method insofar as the processor of the server 100 can obtain information regarding the time. For example, a time specified by the user every day using the mobile information terminal 900m may be transmitted to the server 100. Alternatively, the user's bedtime may be included in the user information input when the account is created.

In addition, an event that had lasted a certain period of time may be estimated as a result of estimation of a plurality of feelings. For example, an event that had lasted a certain period of time, such as a movie, may be estimated on the basis of results of estimation of a plurality of feelings obtained in a certain period of time and event-related data indicating the user's location. The user's feeling on the movie may then be estimated on the basis of a feeling whose intensity was highest while the user was watching the movie and a feeling at an end of the movie. In this case, if the estimated feeling is a feeling of pleasure, content relating to the movie becomes a candidate of content to be presented.

In addition, the example illustrated in Fig. 6 does not limit the configuration of the feeling log database used in the content presentation system 10 according to the present embodiment.

For example, information indicating a category of an event need not be a hashtag. In addition, a type of feeling (feeling type information) and the intensity of the feeling (feeling intensity information) may be stored in the feeling log database instead of, or in addition to, a score indicating a feeling state input to the "feeling state" field.

### 2-3. Operation of Mobile Information Terminal

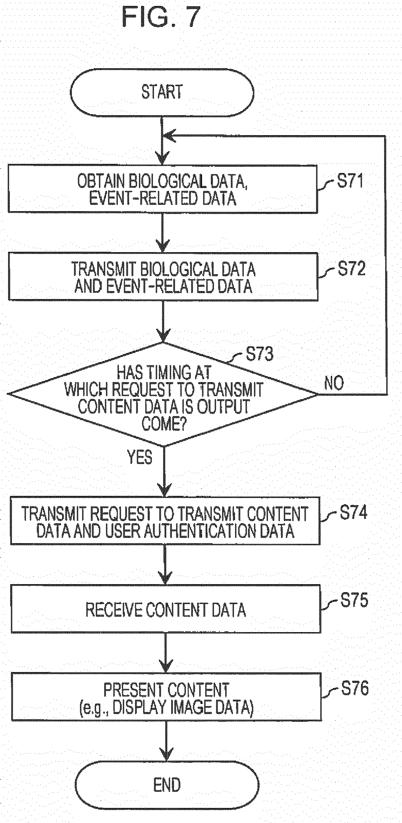

Next, the operation of the mobile information terminal 901 m in the content presentation system 10 will be specifically described with reference to a flowchart of Fig. 7, which illustrates the operation of the mobile information terminal 901m, The following description is a detailed description of the operation of the mobile information terminal 901m, whose outline has been described with reference to Fig. 3.

The mobile information terminal 901m receives and obtains biological data measured and obtained by the biological sensor 920 and obtains event-related data from the positional information obtaining unit 930 and the like (step S71). The mobile information terminal 901m then transmits the obtained biological data and event-related data to the server 100 as necessary (step S72).

Steps S71 and S72 are repeated until a timing at which a request to transmit content data is output (NO in step S73). The timing at which a request to transmit content data is output refers to a timing at which the user performs an operation for requesting transmission of content data. If the user can specify the timing at which a request to transmit content data is output using the mobile information terminal 901m, steps S71 and S72 may be repeated until the timing.

When the timing at which a request to transmit content data is output has come (YES in step S73), the mobile information terminal 901 m transmits the request to transmit content data and user authentication data to the server 100 (step S74).

The mobile information terminal 901m then receives content data from the server 100 (step S75) and presents content to the user using the received content data (step S76). For example, image data is displayed on the mobile information terminal 901 m.

After step S3360 described in section 2-2 as an operation performed by the server 100, the mobile information terminal 901 m may notify the user that content data has been prepared. The notification is performed, for example, after the server 100 notifies the mobile information terminal 901 m that the content data has been prepared or after the content data is received (before step S76). The mobile information terminal 901m uses, for example, a sound, vibration, light, or any selective combination of these for the notification.

### 3. Advantageous Effects

The content presentation system 10 according to the present embodiment includes a biological sensor, a feeling estimation device, an event estimation device, a data generation device, and a terminal device.

The biological sensor measures and obtains biological data regarding a user. In the present embodiment, the biological sensors 920w and 920m, which measure and obtain biological data regarding the user and transmit the biological data to the server 100, which is an information processing apparatus, through the communication network 1000, are examples of the biological sensor. The communication interface 140 of the server 100 receives the biological data, and the storage units 150 store the biological data.

The feeling estimation device estimates a feeling experienced by the user using the biological data. In the present embodiment, the data analysis unit 120, which is achieved by a processor that estimates a feeling using biological data quantified through a certain process, of the server 100, which is the information processing apparatus, is an example of the feeling estimation device.

The event estimation device estimates an event that has evoked the feeling estimated by the feeling estimation device. In the present embodiment, the data analysis unit 120, which is achieved by a processor that estimates an event that has evoked an estimated feeling on the basis of event-related data, of the server 100, which is the information processing apparatus, is an example of the event estimation apparatus.

The data generation device generates content data relating to the estimated pleasurable event and outputs the content data at a certain timing. In the present embodiment, the data generation unit 130, which is achieved by a processor that refers to the feeling log database and generates content data relating to an estimated pleasurable event, of the server 100, which is the information processing apparatus, is an example of the data generation device.

The terminal device presents content to the user using the data output from the data generation device. In the present embodiment, the mobile information terminal 901 m and the information presentation device 900, which receive content data generated by the data generation unit 130 through the communication network 1000 and present content to the user using the content data, are examples of the terminal device used by the user.

The content presentation system 10 configured in this manner is capable of presenting to the user content for helping the user recall events that have evoked feelings of pleasure from the user and prompting the user to make it a habit to recall feelings of pleasure.

The certain timing may be a timing specified by the user in advance. It is recommended to recall feelings of pleasure before a person goes to sleep. The user can recall feelings of pleasure before going to sleep in accordance with his/her daily cycle or his/her schedule of the day. The terminal device capable of presenting content can issue a notification to the user indicating the output of content data. As a result, it becomes less likely for the user to forget to recall feelings of pleasure.

In addition, an event is estimated using, for example, positional information indicating the position of the user, schedule information indicating the user's schedule, image data captured by the user, or the like. The server 100 can obtain these pieces of information, which are examples of information used to estimate an event, directly from the user or through the mobile information terminal 901m.

In addition, in the content presentation system 10, the steps from the estimation of a feeling performed by the server 100 to the presentation of content performed by the terminal device are performed in an active period of the user's daily cycle. Content presented to the user in the presentation of content is content for helping the user recall pleasurable events that have evoked feelings of pleasure in the active period.

As a result, the user makes it a habit to recall feelings of pleasure, that is, recall pleasurable events in an active period, before going to sleep at an end of the active period in the user's daily cycle.

In addition, the biological sensor may measure and obtain biological data regarding the user and the feeling estimation device may estimate the user's feeling on the basis of the biological data while content is being presented to the user. If the user's feeling estimated at this time is a feeling of discomfort, the terminal device may stop presenting the content.

As a result, even if content presented on the basis of an incorrect result of estimation of a feeling evokes a feeling of discomfort from the user, for example, an adverse effect upon the user with respect to the recall of feelings of pleasure is suppressed.

If a plurality of pieces of content data have been generated and there are other pieces of content that have not been presented to the user, the terminal device may present to the user one of the plurality of pieces of content that have not been presented to the user after stopping presenting the first piece of content. In this case, the content presentation system 10 can continue to help the user recall feelings of pleasure.

### Second Embodiment

A content presentation system according to a second embodiment is different from the content presentation system 10 according to the first embodiment in that the content presentation system according to the second embodiment evaluates effects of daily practice of recall of feelings of pleasure and feeds back results of the evaluation by changing a criterion for selecting events to be recalled as a result of presentation of content. The content presentation system according to the present embodiment including such a feedback process will be described hereinafter.

An outline of the content presentation system according to the present embodiment is the same as that of the content presentation system 10 illustrated in Fig. 1, and description thereof is omitted.

### 1. Configuration

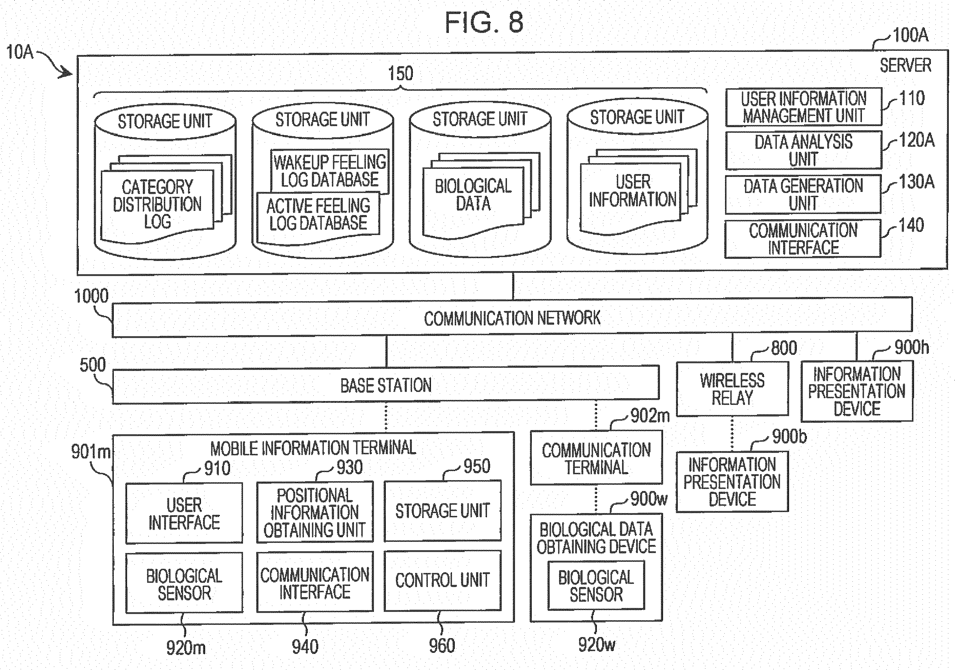

Fig. 8 is a block diagram illustrating the configuration of the content presentation system according to the second embodiment. A content presentation system 10A according to the present embodiment is different from the content presentation system 10 according to the first embodiment in that the content presentation system 10A includes a server 100A instead of the server 100.

The server 100A is different from the server 100 in that the storage units 150 thereof store an active feeling log database, a wakeup feeling log database, and a category distribution log. A data analysis unit 120A and a data generation unit 130A are components provided by a processor included in the server 100A that executes programs. The data analysis unit 120A and the data generation unit 130A perform processes partly different from the processes performed by the data analysis unit 120 and the data generation unit 130, respectively, according to the first embodiment. Other components are the same as those of the server 100, and description thereof is omitted.

The active feeling log database corresponds to the feeling log database according to the first embodiment. The data structure of the active feeling log database is the same as that of the feeling log database illustrated in Fig. 6, and detailed description thereof is omitted.

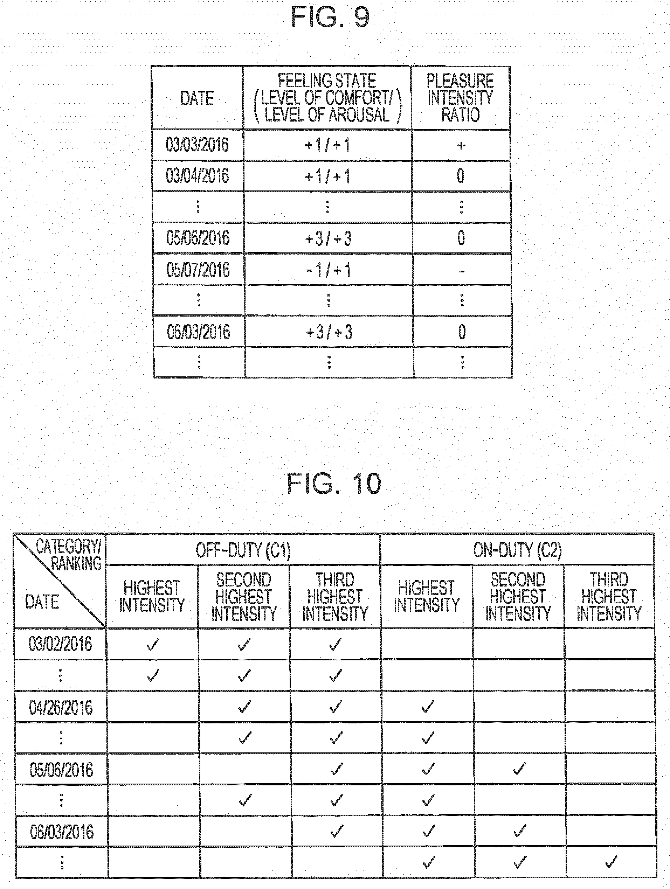

The wakeup feeling log database stores a result of estimation of a feeling performed immediately after the user awakes from his/her sleep, before which the content presentation system 10A presents content. Fig. 9 is a diagram illustrating an example of the data structure of the wakeup feeling log database.

A method for estimating a feeling is basically the same as that used in the first embodiment. First, biological data measured and obtained by the biological sensor 920 is transmitted to the server 100A. The data analysis unit 120A of the server 100A estimates a feeling by analyzing the biological data. As described in the first embodiment, a type of feeling and the intensity of the feeling can be estimated. In the following description, estimation of a feeling includes estimation of both a type of feeling and the intensity of the feeling.

A "feeling state" field of the wakeup feeling log database indicates results (feeling type information and feeling intensity information) of daily analyses of biological data immediately after the user gets up. A "pleasure intensity ratio" field indicates results of comparison between current results and previous results in terms of the intensity of feelings of pleasure. For example, "+" indicates an increase, "-" indicates a decrease, and "0" indicates that there is no change. The intensity of a feeling of discomfort is indicated by a negative value as the intensity of a feeling of pleasure.

Fig. 10 is a diagram illustrating an example of the data structure of the category distribution log.

The category distribution log stores pieces of content selected for presentation from among pieces of content relating to three events that have evoked most intense feelings of pleasure in each category to which pleasurable events can belong. As described in the first embodiment, categories are determined by the data analysis unit 120 on the basis of periods in which pleasurable events have occurred and indicated in the active feeling log database.

The category distribution log illustrated in Fig. 10 indicates that pieces of content were selected as follows:
1. Three pieces of content with checks were presented each day.
2. In each category, pieces of content relating to events that had evoked more intense feelings of pleasure took priority in selection.
3. In an early stage, only pieces of content relating to events belonging to an "off-duty" (C1) category were selected.
4. Pieces of content relating to events belonging to C1 were increasingly less frequently selected, and more pieces of content relating to events belonging to an "on-duty" (C2) category were selected instead. That is, pieces of content for helping the user recall events belonging to "off-duty" took priority over pieces of content for helping the user recall events belonging to the "on-duty" category in the selection of content to be presented to the user.
5. At last, only pieces of content relating to events belonging to C2 were selected.

In the following model case, for example, pieces of content for helping the user recall events belonging to the categories were presented as illustrated in Fig. 10.

A user felt stressed out and depressed from his daily work. In order to control his own feelings by recalling feelings of pleasure, he began to use the content presentation system 10A in March 2016.

Because the user experienced feelings of discomfort when he recalled events that had happened while he was working, initial settings were made such that only pieces of content for helping the user recall pleasurable events belonging to C1 were presented.

On the other hand, the user sometimes experienced a feeling of pleasure such as joy while he was working, and he wanted to have a good time even if he was thinking of his work.

For this purpose, settings were made such that pieces of content for helping the user recall pleasurable events belonging to C2 would also be presented in the future.

On the basis of these settings, first, the content presentation system 10A presented only pieces of content for helping the user recall pleasurable events belonging to C1 and determined whether a desirable effect could be produced by the recall of feelings of pleasure for a certain period. The determination was made on the basis of changes (pleasure intensity ratios) in the intensity of feelings of pleasure immediately after the user got up, which were stored in the wakeup feeling log database.

After it was determined that a desirable effect was produced for the certain period, presentation of a piece of content for helping the user recall a pleasurable event belonging to C2 began (on April 26 in Fig. 10). In this stage, the number of pieces of content presented to help the user recall pleasurable events belonging to C2 was 1. The piece of content was one for helping the user recall a pleasurable event that had evoked a most intense feeling of pleasure belonging to C2 because such a piece of content was expected to produce a desirable effect most easily.

After a desirable effect was observed for a certain period in terms of the control of the user's feelings through the recall of feelings of pleasure with two pieces of content for helping the user recall pleasurable events belonging to C1 and one piece of content for helping the user recall a pleasurable event belonging to C2, the number of pieces of content presented to help the user recall pleasurable events belonging to C1 was decreased, and the number of pieces of content presented to help the user recall pleasurable events belonging to C2 was increased. When the number of pieces of content presented was increased, a piece of content for helping the user recall a pleasurable event that had evoked a more intense feeling of pleasure was selected.

When an adverse effect was observed, the number of pieces of content presented to help the user recall pleasurable events belonging to C2 was decreased, and the number of pieces of content presented to help the user recall pleasurable events belonging to C1 was increased instead (once between May 6 and June 3 in Fig. 10).

That is, the content presentation system 10A evaluated effects produced by the recall of feelings of pleasure and performed feedback by changing the number of pieces of content presented to help the user recall pleasurable events belonging to each category on the basis of the effects.

The operation of the content presentation system 10A that performs such a type of feedback with the above configuration will be described hereinafter.

### 2. Operation

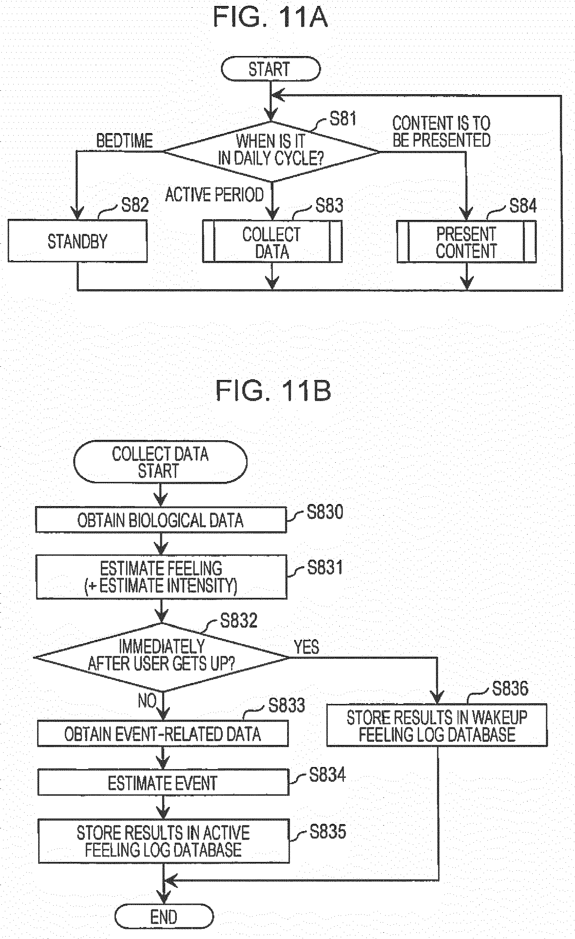

The operation of the content presentation system 10A will be described with reference to Figs. 11A to 11C.

Fig. 11A is a flowchart illustrating an operation cycle of the entirety of the content presentation system 10A.

The operation cycle of the content presentation system 10A follows the user's daily cycle. The user's daily cycle includes an active period, in which the user is awake, and a sleep period, and the user repeats this cycle. The content presentation system 10A repeats the operation cycle in accordance with the user's daily cycle. In order to follow the user's daily cycle, for example, the content presentation system 10A may determine a period from an end of presentation of content to an operation explicitly performed by the user as a sleep period. In addition, in order to determine whether a present time falls within an active period or a sleep period, information regarding the user's daily cycle may be obtained and used as a part of the user information, or one of various pieces of information such as biological data, positional information or schedule information included in event-related data, and a time indicated by an alarm set in the mobile information terminal 901 m may be used. If the biological data obtaining device 900w is smartglasses, for example, it may be determined that a sleep period has ended when it is detected after the sleep period begins that the user has worn the smartglasses.

In step S81, if the present time falls within a sleep period, the content presentation system 10A continues to stand by (step S82). In a standby state, the content presentation system 10A collects data and counts time as necessary for the determination repeatedly made in step S81.

If the present time falls within an active period ("active period" in step S81), the content presentation system 10A collects data (step S83). The collection of data will be described in detail hereinafter with reference to Fig. 11B. Fig. 11B is a flowchart illustrating a procedure of the collection of data performed by the content presentation system 10A.

First, the biological sensor 920 measures and obtains biological data regarding the user (step S830). The obtained biological data is transmitted to the server 100A, and the data analysis unit 120A analyzes the biological data to estimate the user's feeling (step S831). That is, the data analysis unit 120A estimates a type of feeling experienced by the user and the intensity of the feeling.

Next, the data analysis unit 120A determines whether the feeling estimated in step S831 is a feeling immediately after the user gets up (step S832). The determination in step S832 is made on the basis of, for example, whether the user's feeling has been estimated for the first time after a sleep period ends. Alternatively, the user may be asked through the mobile information terminal 901 m to make the determination.

If the estimated feeling is a feeling immediately after the user gets up (YES in step S832), the data analysis unit 120A stores the results of the estimation of a feeling in the wakeup feeling log database (step S836). In the example illustrated in Fig. 9, each of data rows is a record stored through the operation performed by the data analysis unit 120A in step S836.

If the estimated feeling is not a feeling immediately after the user gets up (NO in step S832), the mobile information terminal 901m obtains event-related data including positional information and transmits the event-related data to the server 100A. The data analysis unit 120A of the server 100A analyzes the event-related data to estimate an event (step S834) and stores the event in the active feeling log database along with the results of the estimation of a feeling (step S835). After the data analysis unit 120A stores the results in the wakeup feeling log database (step S836), steps S831 and S832 may be skipped and step S833 may be performed until it is determined next in step S81 that a sleep period has ended.

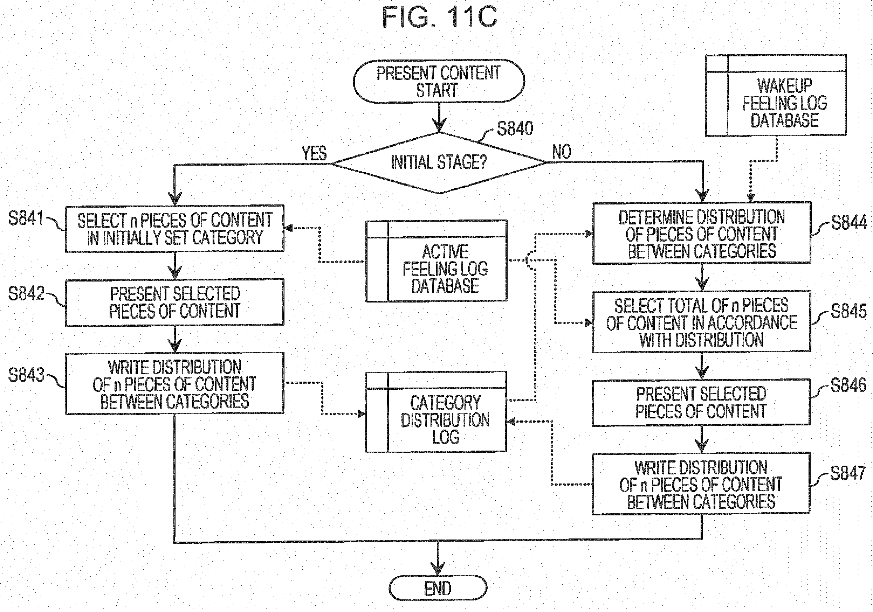

If there is a request to transmit content as described in the first embodiment and the user is successfully authenticated, the content presentation system 10A proceeds to step S84 from step S81. Step S84 will be described in detail hereinafter with reference to Fig. 11C. Fig. 11C is a flowchart illustrating a procedure of presentation of content performed by the content presentation system 10A.

First, the data generation unit 130A of the server 100A determines whether a current use state of the content presentation system 10A of the authenticated user is in an initial stage (step S840). The data generation unit 130A makes the determination, for example, while referring to various logs relating to the use of the content presentation system 10A of the user. Alternatively, the data generation unit 130A may make the determination while referring to information, which is included in the user information, regarding a day on which the user began to use the content presentation system 10A.

If the current use state is in the initial stage (YES in step S840), the data generation unit 130A selects n pieces of content from among pieces of content relating to events belonging to a category specified in initial settings (step S841). Here, n is a natural number indicating the number of pieces of content to be presented, and may be changed in the settings. The category distribution log illustrated in Fig. 10 indicates a case in which n = 3. In the category, the intensity of a feeling of pleasure evoked by each event is referred to, and events that have evoked more intense feelings of pleasure take priority in the selection of events to be recalled. Pieces of content for helping the user recall the events to be recalled then take priority in selection. It is to be noted that the data generation unit 130A refers to the active feeling log database with respect to the category of each event, the intensity of each feeling of pleasure, and a location of each piece of content data.

After generation of data regarding the selected pieces of content is completed, the data generation unit 130A transmits the data to the mobile information terminal 901m or the information presentation device 900. The mobile information terminal 901 m or the like presents the selected pieces of content to the user using the data (step S842). In the above model case, three pieces of content for helping the user recall events to be recalled belonging to C1 were presented to the user from March 2 to April 25 in the procedure in step S841 and later. In other words, in this period, whether to present each piece of content was determined on the basis of whether an event relating to the piece of content belonged to C1 or C2, and pieces of content that could be presented were selected.

In addition, the data generation unit 130A stores the distribution of the n pieces of content between the categories to which events relating to the selected n pieces of content belong in the category distribution log. In the example illustrated in Fig. 10, first and second rows of the data rows are stored through the operation performed by the data generation unit 130A in step S843.

If the current use state is not in the initial stage (NO in step S840), the data generation unit 130A refers to the wakeup feeling log database. The data generation unit 130A then selects a content presentation criterion on the basis of the intensity of a feeling of pleasure immediately after the user gets up (feeling intensity information) included in the wakeup feeling log database. The content presentation criterion specifies the number of events to be recalled for each category (distribution). The data generation unit 130 determines the distribution of the n pieces of content to be presented between the categories in accordance with the selected content presentation criterion (step S844). If the pleasure intensity ratio increases or remains the same for a certain number of times after the user begins to use the content presentation system 10A, for example, the data generation unit 130A selects a content presentation criterion in which the number of events to be recalled belonging to an initially set category is smaller by one and the number of events to be recalled belonging to the other category is larger by one. In the above model case, for example, such a content presentation criterion was selected on April 26. If the pleasure intensity ratio decreases, the data generation unit 130A selects a content presentation criterion in which the number of events to be recalled belonging to the initially set category is larger by one and the number of events to be recalled belonging to the other category is smaller by one. In the above model case, such a content presentation criterion was selected between May 6 and June 3.

Next, the data generation unit 130A selects, in accordance with the distribution of the number of events to be recalled between the categories determined in step S844, the n pieces of content from among the pieces of content for helping the user recall the events belonging to the categories and generates content data regarding the n pieces of content (step S845). In this case, as in step S841, the intensity of feelings of pleasure evoked by the events belonging to each category is referred to, and events that have evoked more intense feelings of pleasure take priority in the selection of the events to be recalled. Pieces of content for helping the user recall the events to be recalled then take priority in selection. It is to be noted that the data generation unit 130A refers to the active feeling log database with respect to the category of each event, the intensity of each feeling of pleasure, and a location of each piece of content data.

After the generation of data regarding the selected pieces of content is completed, the data generation unit 130A transmits the data to the mobile information terminal 901 m or the information presentation device 900. The mobile information terminal 901 m or the like presents the selected pieces of content to the user using the data (step S846). In the above model case, a total of three pieces of content including pieces of content for helping the user recall events to be recalled belonging to C1 and a piece of content for helping the user recall an event to be recalled belonging to C2 were presented to the user from April 26 in the procedure in step S844 and later. In other words, in this period, the number of pieces of content to be presented was determined for each category to which events relating to the pieces of content belong.

In addition, the data generation unit 130A stores the distribution of the selected n pieces of content between the categories in the category distribution log. In the example illustrated in Fig. 10, third and later rows of the data rows are records stored through the operation performed by the data generation unit 130A in step S847.

The content presentation system 10A thus returns to step S81 after the presentation of content (step S84) and stands by until the collection of data (step S83) is performed again (step S82). When the collection of data (step S83) is performed again, the user's feeling immediately after the user gets up is estimated and stored in the wakeup feeling log database (steps S830 to S832 and S836). A content presentation criterion that specifies the number of events to be recalled belonging to each category (distribution) is selected on the basis of the pleasure intensity ratio at this time. This is the feedback process performed by the content presentation system 10A.

In the present embodiment, too, the second feeling estimation step described in the first embodiment may be performed while selected pieces of content are being presented, and if it is determined that the user is experiencing a feeling of discomfort, other pieces of content may be presented. When selecting new pieces of content to be presented, the data generation unit 130A refers to the category distribution log and the active feeling log database as in step S844, and determines pieces of content to be presented to help the user recall events belonging to the categories.

Although the category to which each event belongs is either C1 or C2 in the above description, each event may belong to a plurality of categories as indicated by the "category" field illustrated in Fig. 6, instead.

The operation of the content presentation system 10A has been described. The above description is about an example of the content presentation system 10A, and the operation of the content presentation system 10A according to the present embodiment is not limited to the above description.

In the above description, for example, the intensity of a feeling of pleasure immediately after the user gets up is compared only with the intensity of a previous feeling of pleasure immediately after the user gets up, and a content presentation criterion is selected on the basis of accumulation of results of such comparison in order to determine the distribution of pieces of content to be presented to help the user recall events between the categories. The comparison to be performed is not limited to this example. For example, the intensity of a feeling of pleasure immediately after the user gets up may be compared with an average of intensities of feelings of pleasure immediately after the user gets up in a certain period up to a previous feeling of pleasure. Alternatively, an average of intensities of feelings of pleasure immediately after the user gets up in a certain period including a current feeling of pleasure may be compared with an average of intensities of feelings of pleasure immediately after the user gets up in a certain period up to a previous feeling of pleasure. Alternatively, comparison may be performed once in a certain period, that is, for example, on a certain day in a week, and an average of intensities of feelings of pleasure immediately after the user gets up in a certain week may be compared with an average of intensities of feelings of pleasure immediately after the user gets up in another week. If the intensity of the user's feelings of pleasure does not decrease (remains the same or increases) in this case, content for helping the user recall events in a working period may be selected as content to be presented to the user.

In addition, in the above description, the pleasure intensity ratio, which indicates a change from the intensity of a previous feeling of pleasure, is used for feedback. That is, the intensity of a feeling of pleasure is evaluated on the basis of the intensity of a previous feeling of pleasure. Alternatively, the intensity of a feeling of pleasure may be evaluated on the basis of a certain reference value. The certain reference value may vary depending on how long the content presentation system 10A has been used or the feeling history information regarding the user. For example, the distribution of pieces of content to be presented may be changed if the intensity of a feeling of pleasure is equal to or higher than the certain reference value or if intensities of 10 consecutive feelings of pleasure remain equal to or higher than the certain reference value.

Although whether to present pieces of content and the number of pieces of content to be presented in each category are determined on the basis of a category to which events relating to the pieces of content in the above description, order of presentation of the pieces of content may also be determined for each category. When a plurality of pieces of content are to be presented, for example, a last piece of content tends to leave a strong impression on the user. In view of this, for example, immediately after presentation of a piece of content for helping the user recall an event belonging to the "on-duty" category began in the above model case, the piece of content may be presented first or second among the three pieces of content to be presented. If this piece of content is presented third and the user's feeling state immediately after the user gets up deteriorates (if the intensity of a feeling of pleasure decreases or if the user is experiencing a feeling of discomfort), this piece of content may be presented first or second again.

### 3. Advantageous Effects

The content presentation system 10A according to the present embodiment includes a biological sensor, a feeling estimation device, an event estimation device, a data generation device, and a terminal device.

The biological sensor measures and obtains biological data regarding a user. In the present embodiment, the biological sensors 920w and 920m, which measure and obtain biological data regarding the user and transmit the biological data to the server 100A, which is an information processing apparatus, through the communication network 1000, are examples of the biological sensor. The communication interface 140 of the server 100A receives the biological data, and the storage units 150 store the biological data.

The feeling estimation device estimates, using the biological data, a feeling experienced by the user and the intensity of the feeling as a feeling of pleasure (pleasure intensity). In the present embodiment, the data analysis unit 120A, which is achieved by a processor that estimates a feeling using biological data quantified through a certain process, of the server 100A, which is the information processing apparatus, is an example of the feeling estimation device.

The feeling estimation device also estimates the intensity of a feeling of pleasure after the user to which pieces of content have been presented gets up next time.

The data generation device selects a content presentation criterion on the basis of a result of the estimation of the intensity of a feeling of pleasure immediately after the user gets up, and then selects pieces of content for which data is generated in accordance with the selected content presentation criterion.

A result of the estimation of the intensity of a feeling of pleasure is the user's emotional response to previous presentation of pieces of content, and events to be recalled by the user by presenting next pieces of content are selected using this result as feedback. As a result, it becomes less likely that a piece of content that undesirably makes the user recall an event that adversely affects the user's feeling, and the user can make it a habit to recall feelings of pleasure.

More specifically, for example, each event is classified into one or more categories in advance. Each content presentation criterion specifies the number of events to be recalled for each category (distribution).

The data generation device selects, from among at least one pleasurable event identified in an active period, an event to be recalled by the user by presenting a piece of content in such a way as to satisfy the selected content presentation criterion. The data generation device then generates data regarding a piece of content relating to the selected event to be recalled. In the present embodiment, the data generation unit 130A, which is achieved by a processor that refers to the wakeup feeling log database to select a content presentation criterion and to the active feeling log database to select an event to be recalled and generates a piece of content for helping the user recall the selected event, of the server 100A, which is the information processing apparatus, is an example of the data generation device.

The terminal device presents content to the user using data regarding content output from the data generation device. In the present embodiment, the mobile information terminal 901m and the information presentation device 900, which receive data regarding content generated by the data generation unit 130A through the communication network 1000 and present the content to the user using the data, are examples of the terminal device used by the user.

As a result, for example, a user who is stressed out and feeling uncomfortable during work can try to recall feelings of pleasure in order to feel more comfortable. In an initial stage, events belonging to the "off-duty" category take priority in selection, and the number of events belonging to the "on-duty" category used is gradually increased in later stages in accordance with changes in the user's feeling state caused by presentation of content.

### Third Embodiment

In the content presentation system 10 according to the first embodiment and the content presentation system 10A according to the second embodiment, data obtained through capture of an image, input, or accessed using the mobile information terminal 901 m used by the user or the like is used as a source of data regarding content.

In a content presentation system according to a third embodiment described hereinafter, a source of data regarding content of various types is used The configuration of the content presentation system according to the present embodiment is basically the same as the configuration of the content presentation system 10 or 10A, and illustration thereof is omitted. Components of the content presentation system according to the present embodiment are given the same reference numerals as those of the content presentation system 10 or 10A.

Data (hereinafter also referred to as "external image data" or simply as "external data") regarding content, such as an image, that has been captured and posted on an SNS by a person connected to the user, such as one of the user's family members or friends, but that has not been accessed by the user, for example, may be selected and used.

The data generation unit 130 (or the data generation unit 130A; the same holds in the present embodiment hereinafter) of the content presentation system, for example, obtains such external image data by referring to the feeling log database, obtaining information in the "category" field, and searching an SNS on the basis of the obtained information. In addition, the data generation unit 130 refers to the feeling log database and selects a piece of external image data likely to evoke a feeling of pleasure from the user from among the obtained pieces of external image data. The server 100 then outputs a notification for displaying the selected piece of external image data to the user's terminal device.

As described above, the processor included in the server 100 (or the server 100A; the same holds in the present embodiment hereinafter) achieves the data generation unit 130 by executing a program. Alternatively, the data generation unit 130 may be achieved using techniques of artificial intelligence.

A location of external data is not limited to an SNS, and external data may be obtained from any location insofar as the data generation unit 130 can access the location. If the user's family members and friends are also users (hereinafter referred to as "other users" in the present embodiment) of the content presentation system according to the present embodiment, for example, data regarding content uploaded by the other users and saved to the server 100 may be used as external data.

The user may register the location of external data to the server 100 as a part of the user information to enable the data generation unit 130 to access the external data. For example, the user registers URLs of SNS pages on which content posted by the user's family members and friends, and the data generation unit 130 refers to the user information, obtains the URLs, and accesses the external data.

As in the first and second embodiments, content that can be used to help the user recall feelings of pleasure is selected and presented. As a result, enriched content can be presented even to a user who is busy in an active period and does not have time to capture images or access information uploaded by others, for example, and it becomes possible to help such a user recall feelings of pleasure.

In addition, even when the present embodiment is not used to help a user recall feelings of pleasure, the present embodiment can be applied to fabrication of a system that selects external data likely to evoke feelings of pleasure from the user and presents the external data on a terminal device used by the user. For example, image data (includes external image data) relating to an event that the user or one of the user's family members has experienced may be obtained in accordance with a request issued by the user on his/her way home using the terminal device and presented to the user through the terminal device.

In addition, the user may perform, using the terminal device, an operation for expressing his/her reaction, such as selection or evaluation, to image data presented in this manner, and the terminal device may transmit information indicating the user's reaction to the server 100. The data generation unit 130 of the server 100 generates data regarding content to be presented to the user before the user goes to sleep on the basis of the information indicating the user's reaction.

### Fourth Embodiment

In a content presentation system according to a fourth embodiment that will be described hereinafter, when data regarding content to be presented to the user is generated, the feeling log database including results of estimation of feelings of users other than the foregoing user is referred to. The phrase "users other than the foregoing user" refers to, for example, the user's family members, friends, and the like who are likely to sympathize with the user. The configuration of the content presentation system according to the present embodiment is basically the same as that of the content presentation system 10 or 10A, and illustration thereof is omitted. Components of the content presentation system according to the present embodiment are given the same reference numerals as those of the content presentation system 10 or 10A.

More specifically, when the user's (hereinafter referred to as a "user in question") family members and friends are also users (hereinafter referred to as "other users") of the content presentation system, for example, the data analysis unit 120 (or the data analysis unit 120A; the same holds in the present embodiment hereinafter) of the content presentation system estimates the other users' feelings in certain periods using the same method as in the estimation of the user's feeling in the first embodiment. That is, the communication interface 140 receives biological data or the like measured and obtained from the other users from terminal devices, such as mobile information terminals 901 m, used by the other users. The data analysis unit 120 estimates the other users' feelings using the biological data or the like. Results of the estimation of the other users' feelings are associated with events estimated to have evoked the feelings and stored in feeling log databases for the other users (other user databases). The feeling log databases for the other users store the certain periods (other user certain periods), feeling state information regarding the feelings experienced by the other users (other user feeling type information and other user feeling intensity information), and information indicating the estimated events (other user event information), which are associated with one another. The certain periods of the other users (other user certain periods) and the certain period of the user in question may be the same or different from each other.

If the other user feeling type information included in records (other user feeling history information) of the feeling log databases for the other users indicates feelings of pleasure, the data analysis unit 120 determines that the events indicated by the records are likely to evoke feelings of pleasure from the user in question.

The data analysis unit 120 then enables the data generation unit 130 (or the data generation unit 130A; the same holds in the present embodiment hereinafter) to use the information regarding the records included in the feeling log databases for the other users when the data generation unit 130 obtains and generates data regarding content to be presented to the user in question later (step S335). More specifically, if there are records in the feeling log database for the user in question including information regarding events corresponding to the above records (determined on the basis of times, places, and categories), for example, the other user event information (the "category" field, the "content data source" field, or both) may be copied to the records in the feeling log database for the user in question. If there are no records including information regarding events corresponding to the above records in the feeling log database for the user in question, all the records of the other users may be copied. As a result, other user image data indicating the other users' events can be generated using the other user event information associated with the other user feeling type information indicating feelings of pleasure. The other user image data can then be transmitted to the terminal device used by the user in question and presented to the user in question. In the present embodiment, too, a notification for displaying the other user image data as image data for the user in question may be output as in the first embodiment.

As a result, enriched content can be presented to the user in question as content for helping the user in question recall pleasurable events experienced by the user in question together with the other users.

Although whether an event indicated by each record is likely to evoke a feeling of pleasure from the user in question is determined on the basis of whether the other user feeling type information included in the record indicates a feeling of pleasure, the determination need not be made in this manner. If category information included in each record includes information indicating the user in question, for example, it may be determined that the event is likely to evoke a feeling of pleasure from the user in question, instead. In this case, other user image data indicating events that have evoked feelings of pleasure from the other users but are unrelated to the user in question can be excluded from candidates for image data to be presented to the user in question, and privacy of the other users is protected.

In addition, event information or records copied from the feeling log databases for the other users may be given flags indicating that the event information or the records are copies. Content based on the copied event information or records may be given information indicating that the content is based on the copied event information or records and presented to the user in question.

In addition, although all the records of the other users are copied to the feeling log database for the user in question if there are no records including event information included in a record of another user in the feeling log database for the user in question, the record need not be copied. The information included in the record might be information that the other user wants to tell the user in question about directly.

In addition, in the present embodiment, data regarding content can be automatically shared between users without a user making a determination for each event or each piece of content. In consideration of protection of privacy, therefore, the above-described relationship between the user in question and the other users is desirably established only under certain conditions, such as when the user in question and the other users have agreed through the user information.

### Modifications

In the above description of the content presentation systems according to the embodiments, the user's involvement in the presentation of content is not described. The presentation of content, however, may be interactively performed with the user.

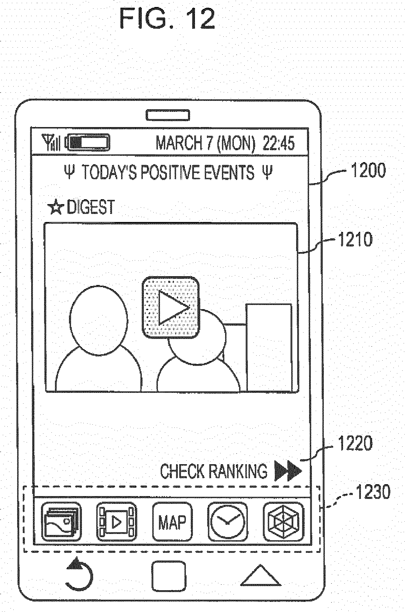

Fig. 12 is a diagram illustrating an example of an operation screen displayed in order to present content in each embodiment.

An operation screen 1200 includes an image 1210 before a moving image is played back, a link 1220 to a ranking of a selected piece of content, and icons 1230 as objects that the user can actively operate. Each of the objects is used to present content to the user in a different manner.

If the user touches the image 1210, for example, a digest of a piece of content selected in each of the content presentation systems is played back. Data regarding the digest is an example of data generated by the data generation unit 130 or 130A. Order of presentation of a plurality of pieces of content is fixed in the digest.

If the user touches the link 1220, a list in which pieces of image data regarding content selected in each of the content presentation systems are arranged in certain order, such as a list in which pieces of image data are arranged in descending order of intensity of feelings of pleasure evoked by events to be recalled, is displayed. The user selects an arbitrary piece of image data in the list to display or play back a corresponding piece of image data.

The data generation unit 130 or 130A of the server 100 or 100A refers to the feeling log database to generate such a list, for example, and the server 100 or 100A may output a notification for displaying the list to the terminal device used by the user. Alternatively, the terminal device used by the user may generate the list on the basis of data regarding content received from the server 100 or 100A.

If the user touches one of the icons 1230, a list of pieces of content is displayed in another manner. In the example illustrated in Fig. 12, the icons are used to display, from left to right, a list of still images, a list of moving images, a map indicating places where events have occurred, a list of pieces of content in chronological order, and a radar chart based on types of feelings and the intensity of the feelings.

These are just examples, but since the user can display a list in a way that he/she desires, the user can enjoy the recall of feelings of pleasure before going to sleep and make it a habit to recall feelings of pleasure.

A screen displayed in order to present content is not limited to the screen illustrated in Fig. 12 that includes many options. For example, the user may select one of the options in user settings, and pieces of content may be displayed to the user in a selected manner.

Such an operation screen may be provided for the user, for example, by an application dedicated to the system installed on the mobile information terminal 901m or the information presentation device 900, or may be provided for the user as a webpage displayed in a common web browser.

Although the content presentation system according to one or a plurality of aspects have been described on the basis of the above embodiments, the present disclosure is not limited to the above embodiments. The scope of the one or plurality of aspects may include modes obtained by modifying the above embodiments in various ways conceivable by those skilled in the art and modes constructed by combining components in different embodiments with each other without deviating from the spirit of the present disclosure.

In addition, the present disclosure may be implemented as a method including the steps performed by the components in one of the above embodiments.

In addition, the present disclosure may be implemented as one or more server apparatuses including a communication interface for communicating with a biological sensor that obtains data from a user and a terminal device that presents content to the user, a feeling estimation device, an event estimation device, and a data generation device.

Although the feeling estimation device, the event estimation device, and the data generation device are included a server that communicates with the biological sensor and the terminal device through a communication network in the above description of the content presentation system according to the one or plurality of aspects, the present disclosure is not limited to this. Some or all of the feeling estimation device, the event estimation device, and the data generation device may be included in a device worn or carried by the user in which the biological sensor and the terminal device are included, instead. In this case, the feeling estimation device is a component achieved by a processor, which is included in a mobile information terminal carried by the user, that executes a program for analyzing biological data and estimating a feeling, for example, and obtains biological data from the biological sensor without using the communication network. In addition, the event estimation device is a component achieved by a processor, which is included in the mobile information terminal carried by the user, that executes a program for analyzing event-related data and estimating an event, for example, and obtains data used by another program in the mobile information terminal as event-related data.

The present disclosure can be used as a system and a method that present content to help a user control his/her feelings on the basis of psychology.

## Claims

1. An information processing apparatus comprising:
a communication interface that receives biological data obtained from a user;
a memory that stores the biological data received by the communication interface; and
a processor that determines a type of feeling experienced by the user in a certain period using the biological data,
wherein the processor estimates an event that has occurred to the user in the certain period, stores, in a database, feeling history information in which the certain period, feeling type information indicating the type of feeling, and event information indicating the estimated event are associated with one another, generates, if the feeling type information stored in the database indicates a feeling of pleasure, image data for helping the user recall the event using the event information associated with the feeling type information, and outputs a notification for displaying the image data to a terminal device used by the user.

2. The information processing apparatus according to Claim 1,
wherein the processor obtains information indicating the user's bedtime set by the user and, when the user's bedtime has come, outputs the notification to the terminal device.

3. The information processing apparatus according to Claims 1 or 2,
wherein the processor obtains information for identifying the user's working period and gives priority to image data for helping the user recall an event that has occurred outside the user's working period over image data for helping the user recall an event that has occurred in the user's working period in the outputting to the terminal device.

4. The information processing apparatus according to Claim 3,
wherein the user's feeling is estimated on the basis of biological data regarding the user measured after the user watches, on the terminal device, the image data for helping the user recall an event that has occurred outside the user's working period for a certain period, and
wherein, if the user's feeling does not decrease after the certain period in terms of a feeling of pleasure, image data for helping the user recall an event that has evoked a feeling of pleasure in the user's working period is output to the terminal device.

5. The information processing apparatus according to any of Claims 1 to 4,
wherein the processor refers to the database, creates a list of pieces of content arranged in order of intensity of feelings of pleasure evoked by events relating to image data to be presented, outputs a notification for displaying the list of the pieces of content to the terminal device, and, after the user selects a piece of image data included in the list of the pieces of content, displays the piece of content on the terminal device.

6. The information processing apparatus according to Claim 1,
wherein the processor obtains external image data uploaded by another user, who is a user other than the foregoing user, to a location accessible by the processor, refers to the database, selects a piece of the external image data likely to evoke a feeling of pleasure from the user, and outputs a notification for displaying the selected piece of the external image data to the terminal device used by the user.

7. The information processing apparatus according to Claim 1,
wherein the communication interface receives biological data regarding another user, who is a user other than the foregoing user,
wherein the memory stores the biological data regarding the other user received by the communication interface, and
wherein the processor determines a type of feeling experienced by the other user in a certain period, estimates an event that has occurred to the other user in the certain period, stores, in a database, feeling history information in which the certain period, feeling type information indicating the type of feeling experienced by the other user, and event information indicating the event that has occurred to the other user are associated with one another, generates, if the feeling type information regarding the other user stored in the database indicates a feeling of pleasure, image data indicating the event that has evoked the feeling of pleasure from the other user using the event information associated with the feeling type information regarding the other user, and displays the image data on the terminal device used by the foregoing user.

8. The information processing apparatus according to Claim 1,
wherein the processor performs the determination as to a type of feeling, the estimation of an event, the storing in the database, the generation of image data, and the outputting of a notification to the terminal device in an active period, which is a period in which the user is awake, and
wherein the generated image data is displayed to help the user recall an event associated with feeling type information indicating a feeling of pleasure in the database among events identified in the active period in which the generation is performed.

9. The information processing apparatus according to Claim 8,
wherein the processor also estimates, in the active period, intensity of the user's feeling of pleasure on the basis of biological data regarding the user obtained immediately after the user gets up, selects a content presentation criterion on the basis of the estimated intensity of the user's feeling of pleasure, selects, from among at least one pleasurable event identified in the active period, an event to be recalled by the user by displaying the image data such that the selected content presentation criterion is satisfied, and generates, as the image data, image data for helping the user recall the selected event to be recalled.

10. The information processing apparatus according to Claim 9,
wherein the processor also classifies the estimated event into a category, refers to the selected content presentation criterion, which specifies a number of events to be recalled for each category, selects, from among the at least one pleasurable event identified in the active period, events to be recalled corresponding to the number of events to be recalled specified for each category in the selected content presentation criterion on the basis of the category into which the estimated event has been classified, and generates image data for helping the user recall the selected events to be recalled.

11. The information processing apparatus according to any of Claims 1 to 10,
wherein the processor also determines a type of feeling that is being experienced by the user on the basis of biological data regarding the user obtained while the image data is being presented to the user and, if the determined type of feeling is a feeling of discomfort, stops presenting the image data to the user.

12. The information processing apparatus according to Claim 11,
wherein, if a plurality of pieces of the image data are generated, the processor also selects, after stopping presenting the image data to the user, one of the plurality of pieces of the image data other than the image data that has been presented and presents the selected piece of the image data to the user.

13. A method for controlling an information processing apparatus, which performs operations comprising:
receiving biological data obtained from a user through a communication network;
storing the received biological data in a memory;
determining a type of feeling experienced by the user in a certain period using the received biological data;
estimating an event that has occurred to the user in the certain period;
storing, in a database, feeling history information in which the certain period, feeling type information indicating the type of feeling, and event information indicating the estimated event are associated with one another;
generating, if the feeling type information stored in the database indicates a feeling of pleasure, image data for helping the user recall the event using the event information associated with the feeling type information; and
outputting a notification for displaying the image data to a terminal device used by the user.

14. A non-transitory recording medium storing a program, which causes a computer to perform operations comprising:
receiving biological data obtained from a user through a communication network;
storing the received biological data in a memory;
determining a type of feeling experienced by the user in a certain period using the received biological data;
estimating an event that has occurred to the user in the certain period;
storing, in a database, feeling history information in which the certain period, feeling type information indicating the type of feeling, and event information indicating the estimated event are associated with one another,
generating, if the feeling type information stored in the database indicates a feeling of pleasure, image data for helping the user recall the event using the event information associated with the feeling type information; and
outputting a notification for displaying the image data to a terminal device used by the user.
